# EUROPEAN PATENT APPLICATION

(11) **EP 4 545 651 A1**
(43) Date of publication of application: **30.04.2025**
(21) Application number: 23205776.0
(22) Date of filing: 25.10.2023
(51) Int. Cl.: C12Q 1/6883

(54) **NOVEL BIOMARKERS FOR PROGNOSIS OF COMMUNITY ACQUIRED PNEUMONIA (CAP)**

(71) Applicant: Charité - Universitätsmedizin Berlin, 10117 Berlin (DE); Universität Leipzig, 04107 Leipzig (DE); Universitätsklinikum Jena, 07747 Jena (DE); Justus-Liebig-Universität Gießen, 35390 Gießen (DE); Medizinische Hochschule Hannover, 30625 Hannover (DE)
(72) Inventor: Schütte, Hartwig, 10405 Berlin (DE); Suttorp, Norbert, 16540 Hohen Neuendorf (DE); Creutz, Petra, 14163 Berlin (DE); Kiehntopf, Michael, 07749 Jena (DE); Bauer, Michael, 07749 Jena (DE); Kirsten, Holger, 04318 Leipzig (DE); Scholz, Markus, 04425 Taucha (DE); Löffler, Markus, 04229 Leipzig (DE); Ahnert, Peter, 04109 Leipzig (DE); Chakraborty, Trinad, 35394 Gießen (DE); Welte, Tobias, 30625 Hannover (DE)
(74) Representative: Schulz Junghans Patentanwälte PartGmbB

(57) **Abstract**

The present invention refers to a diagnostic method for the ex-vivo prognosis, prediction or risk stratification for mortality, disease outcome or ICU admission of a patient who has been diagnosed with community acquired pneumonia (CAP) comprising the steps of i) providing a sample from the patient and ii) analysing the expression level of at least one nucleic acid in the sample, wherein the patient's prognosis, prediction or risk stratification is determined according to the expression levels of the at least one nucleic acid. The at least on nucleic acid is selected from a gene signature comprising biomarkers as identified in the present invention. In further aspects the present invention also refers to the nucleic acids of the gene signature and their use for prognosis and risk stratification. Finally, the present invention also refers to a diagnostic kit for performing the method of the invention

## Description

### Field of the invention

The present invention is in the field of biology and chemistry. In particular, the invention is in the field of molecular biology. More particularly, the invention relates to the analysis of RNA transcripts. Most particularly, the invention is in the field of diagnosis and prognosis of community acquired pneumonia (CAP).

### Background of the invention

Pneumonia is among the leading causes of morbidity and mortality worldwide. Each year, approximately 660,000 patients in Germany alone are diagnosed with community-acquired pneumonia (CAP), i.e. pneumonia that develops outside of hospitals or other health care settings, of which almost half will be admitted and 13% will die within 30 days (Kolditz et al. (2016)) resulting in about 29,000 deaths in the context of CAP. CAP patients are at high-risk for rapid disease deterioration. In fact, every fifth CAP patient admitted to the hospital will require treatment in an intensive care unit (ICU) and mortality among those patients remains high (Gearhart et al. (2019), Jain et al. (2015)). Delayed admission to the ICU of CAP patients is a risk factor for excess mortality (Renaud et al. (2012), Restrepo et al. (2010)). In Germany, this affects approximately a third to half of CAP patients requiring ICU treatment. Given the approximately 20% higher mortality rate for late ICU transfer, it can be estimated that an improved risk assessment could save up to a four-digit number of lives in Germany alone. Hence, (CAP) is a severe, common disease with a still high mortality. Thus, early risk stratification, i.e. upon admission, carries potential to improve care and ultimately survival (Gearhart et al. (2019)). A biomarker, i.e. an objective signal that can be measured reproducibly and accurately, allowing for early detection of a potentially severe disease course, could provide invaluable assistance to the clinician and patient alike (Dela Cruz et al. (2018)).

Recently, the inventors have shown that the Sequential Organ Failure Assessment (SOFA) score reflects CAP severity on the day of hospital admission better than many other clinically employed scores (Ahnert et al. (2019)). However, this only means that the SOFA score is adequate to distinguish patients with a severe course of CAP from patients with a normal course of CAP. Moreover, several studies have investigated biomarkers to predict severe courses of CAP, though their clinical utility in a general setting remains to be proven (Sibila et al. (2019), Torres et al. (2019)). For example, routine clinical measurements, such as C-reactive protein or interleukin-6, are inferior to American Thoracic Society and the Infectious Diseases Society of America (IDSA/ATS) criteria for predicting ICU admission (Ramírez et al. (2011)), Other clinical measures, such as CURB65 predicts 30-day mortality, but fail to indicate ICU requirement (Torres et al. (2019)). Therefore, unfortunately there are not yet sufficiently good prognostic parameters for identifying patients who are unremarkable at the time of measurement but will develop such a severe course in the future

So far, treatment of CAP patients upon admission is performed according to clinical guidelines (e.g., immediate antibiotic administration) and physician's decision on whether patient is admitted to normal ward or directly to ICU. On ICU patients are closely monitored which includes advanced blood analysis, monitoring of temperature, oxygen saturation, etc. Contrary, on normal wards, usually only blood analysis of general clinical values and no close monitoring is performed.

Gene expression analysis from blood samples presents an opportunity to detect severe progression of CAP before clinical manifestation. Although transcriptome-based multiplexed biomarkers for sepsis, encompassing a large subgroup of patients with a pulmonary disease, are available, these profiles were typically developed in cohorts already admitted to the ICU (Scicluna et al. (2017), Sweeney et al. (2018), Tsalik et al. (2014), Wong et al. (2015)). Molecular predictors have been proven as useful in discriminating CAP vs. non-CAP patients (Scicluna et al. (2015)), CAP vs. with hospital-acquired pneumonia (van Vught et al. (2016)), sterile inflammation vs. sepsis (Sweeney et al. (2015)), inflammation severity in sepsis (Bauer et al. (2016)), and bacterial vs. viral infection (Sweeney et al. (2016)). Moreover, certain signatures serve as indicators for overall disease severity (Almansa et al. (2015)). However, despite the obvious clinical need, means to predict deterioration of organ function early on in the disease course of CAP are lacking.

The present invention is based on an unbiased analysis of whole blood transcriptome data from hospitalized patients with CAP to the aim of identifying a gene expression signature that can predict the disease course immediately upon admission.

### Brief description of the invention

In a first aspect the present invention refers to a diagnostic method for the ex-vivo prognosis, prediction or risk stratification for mortality, disease outcome or ICU admission of a patient who has been diagnosed with community acquired pneumonia (CAP) comprising the steps of
a) providing a sample from the patient,
b) analysing the expression level of at least one nucleic acid in the sample,
   wherein the patient's prognosis, prediction or risk stratification is determined according to the expression levels of the at least one nucleic acid,
   wherein the at least one nucleic acid is selected from the group comprising CLEC4A, KLRB1, SIGLEC14, TNFSF14 and YOD1.

Another aspect of the invention is a nucleic acid that hybridizes under stringent conditions to one of the nucleic acids according to SEQ ID NO: 1-13, or the reverse complement thereof or any part thereof, or a nucleic acid that shares preferably at least 85%, 90%, 95% or 99% sequence identity with one of the sequences of SEQ: 1-13, or the reverse complement thereof or any part thereof. The nucleic acids of the invention may be used for determining mRNA expressions levels of the biomarker genes of the gene signature of the invention. The nucleid acids of the invention are listed in the sequence listing and table 7.

Another aspect of the present invention refers to the use of the nucleic acid according to the method of the invention for the prognosis, prediction or risk stratification for mortality, disease outcome or ICU admission of a patient who has been diagnosed with community acquired pneumonia (CAP).

Another aspect of the invention refers to a diagnostic kit for the prognosis, prediction or risk stratification for mortality, disease outcome or ICU admission of a patient who has been diagnosed with community acquired pneumonia (CAP), wherein the kit comprises:
a) a nucleic acid according to SEQ ID NO: 1-13;
b) reagents for nucleic acid amplification and/or quantification and/or detection.
c) optionally standards and/or internal standards and
d) reference data for patients under reference conditions,
e) a manual.

The kit of the invention is diagnostic kit for a quick bedside examination of the patient with CAP for direct risk stratification and prognosis of the patient upon hospital admission. The components of the kit are optimized for automation of the assay.

### Definitions

The following definitions are provided for specific terms, which are used in the application text.

Unless otherwise defined, all technical and scientific terms used herein have the same meaning as commonly understood by one skilled in the art to which this technology belongs. Although all methods and materials similar or equivalent to those described herein may be used in practice or in testing the present technology, the preferred methods, devices and materials are now described.

Where an indefinite or a definite article is used when referring to a singular noun such as "a" or "an" or "the", this includes a plural form of that noun unless specifically stated. Vice versa, when the plural form of a noun is used it refers also to the singular form. For example, when biomarkers are mentioned, this is also to be understood as a single biomarker.

As used herein, "nucleic acid(s)" or "nucleic acid molecule" generally refers to any ribonucleic acid or deoxyribonucleic acid, which may be unmodified or modified. "Nucleic acids" include, without limitation, single- and double-stranded nucleic acids. As used herein, the term "nucleic acid(s)" also includes nucleic acids as described above that contain one or more modified bases. Thus, a nucleic acid with one or several backbone modifications for stability or for other reasons is a "nucleic acid". The term "nucleic acids" as it is used herein encompasses such chemically, enzymatically or metabolically modified forms of nucleic acids, as well as the chemical forms of nucleic acids characteristic of viruses and cells, including for example, simple and complex cells.

The term "transcript" relates to a nucleic acid produced by making a copy of a template nucleic acid. Included in this definition is the nucleic acid obtained through transcription by a polymerase of a template nucleic acid, for example a locus, to produce the new nucleic acid with a sequence complementary to the template. The term transcript also includes any nucleic acid further processed after transcription. Such further processing includes, but is not limited to, splicing, polyadenylation, editing, partial digestion, ligation, and labelling. The term also encompasses nucleic acids derived from the transcript through for example further transcription, reverse transcription, amplification or elongation. The transcript can correspond to any portion of the template nucleic acid, preferably to at least 20 nucleotides. The transcript also has a sequence identity with the template nucleotide or part of the template nucleotide, or the reverse complement of the template nucleic acid of at least 90%, preferably at least 95% and most preferably at least 99%.

"Sample molecules" or "individual sample templates" as used herein refers to any kind of nucleic acid molecules contained in a sample to be analysed, such as single-stranded or double-stranded DNA and/or RNA.

"Biomarker" within the present invention refers to any gene, gene transcript, gene transcript variant or non-coding genomic sequence which are predictive for prognosis, prediction or risk stratification for mortality, disease outcome or ICU admission of a patient who has been diagnosed with community acquired pneumonia (CAP).

The terms "level" or "expression level" in the context of the present invention relate to the level at which a biomarker is present in a sample from a patient. The expression level of a biomarker is generally measured including a normalization step, e. g. quantile normalization or by comparing its measured signal to the measured signal of one or several reference or housekeeping genes in a sample. From the respective expression level data of the seleceted biomarkers, which may be log2-transformed, a score is calculated which may or may not be combined with another clinical score to form a combined prognositc score. The calculated score is then used for prognosis by comparison to reference values.

The term, "analysing a sample for the presence and/or level of nucleic acids" or "specifically estimate levels of nucleic acids", as used herein, relates to the means and methods useful for assessing and quantifying the levels of nucleic acids. Several different useful method are known to the skilled person in the field for instance quantitative reverse transcription PCR, TaqMan assays, lonTorrent, Microarrays and sequencing techniques. Likewise, the level of RNA can also be analysed for example by northern blot, next generation sequencing or after amplification by using spectrometric techniques that include measuring the absorbance at 260 and 280 nm or measuring the specific fluorescence of various fluorescent dyes which detect nucleic acids, in particular intercalating fluorescent dyes.

The "amplification" of the individual sample templates refers to any kind of nucleic acid amplification method which results in the generation of multiples of the original template.

Analysis or examination herein refers to identifying whether or not amplification has taken place, identifying whether or not the target sequence lies between the primer regions and optionally, has the right length, identifying the amount of amplification product with a correct target sequence. Preferably in the method of the invention precise quantification of the amplification product is desired.

As used herein, the term "amplified", when applied to a nucleic acid sequence, refers to a process whereby one or more copies of a particular nucleic acid sequence is generated from a nucleic acid template sequence, preferably by the method of polymerase chain reaction (PCR). Other methods of amplification include, but are not limited to, ligase chain reaction (LCR), polynucleotide-specific based amplification (NSBA), loop-mediated isothermal amplification (LAMP), quantitative reverse transcription real-time PCR (qRT-PCR), quantitative (real-time) PCR (qPCR), droplet digital PCR, digital PCR, or any other amplification method known in the art.

The term "correlating", as used herein in reference to the use of diagnostic and prognostic marker(s), refers to comparing the presence or amount of the marker(s) in a sample from a patient to its presence or expression level in a sample from a person known to suffer from or at risk of suffering from a given condition. A marker expression level in a patient sample can be compared to a level known to be associated with a specific diagnosis.

As used herein, the terms "diagnosis" or "diagnostic" refer to the identification of a disease in a subject, at any stage of its development, and also includes the determination of predisposition of a subject to develop the disease. The method of the invention concerns prognosis, prediction or risk stratification for mortality, disease outcome or ICU admission of a patient who has been diagnosed with community acquired pneumonia (CAP). This means the prognosis of a severe disease, the need for CAP-specific intensive care or a high risk of death.

The "labeling" of the DNA or RNA strands can be realized by associating or incorporating any kind of marker detectable by conventional imaging techniques, e.g. a fluorescent marker.

As used herein, the term "fluorescent dye" refers to any chemical that absorbs light energy of a specific wavelength and re-emits light at a different wavelength.

Application of a fluorescence dye for target detection implies either use of intercalating dyes in a PCR reaction to detect formation of double-stranded DNA molecules, or labelling primers or probes with suitable fluorophore molecules to observe presence of formation of nucleic acid molecules with complementary target sequences in dependence on the detected fluorescence signals of the fluorophore molecules. By using different fluorophores with distinct emission and excitation spectra it is possible to combine more than one detection system into one PCR reaction (multiplex PCR) and to separately detect the fluorescence signals. Potential labelling molecules include but are not limited to fluorescence dye or chemiluminescence dye in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like. In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562, incorporated herein by reference, including citations on pages 551-562. Preferred chemiluminescent dyes are acridiniumesters.

As used herein, "isolated" when used in reference to a nucleic acid means that a naturally occurring sequence has been removed from its normal cellular (e.g. chromosomal) environment or is synthesised in a non-natural environment (e.g. artificially synthesised). Thus, an "isolated" sequence may be in a cell-free solution or placed in a different cellular environment.

As used herein, a "kit" is a packaged combination optionally including instructions for use of the combination and/or other reactions and components for such use. If the kit contains nucleic acids, the kit may also comprise synthetic or non-natural variants of said nucleic acids. A synthetic or non-natural nucleic acid is to be understood as a nucleic acid comprising any chemical, biochemical or biological modification, such that the nucleic acid does not appear in nature in this form. Such modifications include, but are not limited to, labelling with a fluorescent dye or a quencher moiety, a biotin tag, as well as modification(s) in the backbone of a nucleic acid, or any other modification that distinguishes the nucleic acid from its natural counterpart. The same applies also to other natural compounds such as proteins, lipids and the like.

The term "patient" as used herein refers to a living human or non-human organism that is receiving medical care or that should receive medical care due to a disease, or is suspected of having a disease.

The term "primer" as used herein, refers to an nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. Preferably, primers have a length of from about 15-100 bases, more preferably about 20-50, most preferably about 20-40 bases. The factors involved in determining the appropriate length of primer are readily known to one of ordinary skill in the art. Optionally, the primer can be a synthetic element, in the sense that it comprises a chemical, biochemical or biological modification. Such modifications include, but are not limited to, labelling with a fluorescent dye or a quencher moiety, or a modification in the backbone of a nucleic acid, or any other modification that distinguishes the primer from its natural nucleic acid counterpart.

The term "probe" refers to any element that can be used to specifically detect a biological entity, such as a nucleic acid, a protein or a lipid. Besides the portion of the probe that allows it to specifically bind to the biological entity, the probe also comprises at least one modification that allows its detection in an assay. Such modifications include, but are not limited to labels such as fluorescent dyes, quenchers, a specifically introduced radioactive element, or a biotin tag. The probe can also comprise a modification in its structure, such as a locked nucleic acid.

The term "sample" as used herein refers to a sample of bodily fluid or tissue obtained for the purpose of diagnosis, prognosis, or evaluation of a subject of interest, such as a patient. In addition, one of skill in the art would realize that some test samples would be more readily analysed following a fractionation or purification procedure, for example, separation of whole blood into serum or plasma components.

Thus, in a preferred embodiment of the invention the sample is selected from the group comprising blood, whole blood, blood serum, blood plasma, buffy coat, urine, sputum, tracheal secretions, cheek swabs and tissue samples and including other bodily fluids and secretions. Preferably, the sample is blood, whole blood or buffy coat. blood sample, most preferably a serum sample or a plasma sample. The samples may or may not be treated to stabilize and preserve RNA.

The term "area under the curve (AUC)" as used herein describes the area under the curve of a receiver operating characteristic (ROC) or ROC curve. The AUC relates to how specific and sensitive a biomarker is. A perfect marker (AUC=1.0) would yield a point in the upper left corner or coordinate (0,1) of the ROC space, representing 100% sensitivity (no false negatives among all true positives) and 100% specificity (no false positives among all true negatives). Similarly, the term "area und the precision recall curve" (AUC-PR) describes the area under the curve defined by precision vs. recall. Here, a perfect marker (AUC-PR=1) would yield a point in the upper rigth corner or coordinate(1,1) of the plot, representing 100% precision (no false positives among all identified positives) and 100% recall (same as sensitivity, i.e. no false negatives among all true positives).

If not stated otherwise, AUC ROC value and AUC-PR values are linearly scaled in the following text to a range between 0 and 100, where a perfect AUC of the ROC analysis of 1.0 and a perfect AUC-PR are scaled to the value 100, respectively.

The term "p-value" relates to the probability of obtaining the observed sample results (or a more extreme result) when the null hypothesis is actually true, i.e. there are no differences between means for groups. The smaller the p-value, the higher the likelihood that the alternative hypothesis explains the observed results better than the null hypothesis.

The term "sensitivity" or "recall" of a medical test within the present application refers to a test's ability to designate an individual with disease as positive. A highly sensitive test means that there are few false negative results, and thus fewer cases of disease are missed.

The term "specificity" of a medical test within the present application refers to a test's ability to designate an individual who does not have a disease as negative. A highly specific test means that there are few false positive results.

The term "precision" or "positive predictive value" (PPV) of a medical test within the present application refers to a test's reliability that a predicted positive sample actually was obtained from a subject who has the disease. A highly precise test means that there are few false positve results.

A "severe disease course" is defined within this application based on a composite endpoint (cEP) consisting of i.) 28-day all-cause mortality or ii.) qualified admission to the ICU, where qualified admission was defined as treatment with substantial respiratory support (ventilation, extracorporeal oxygenation, or oxygen supplementation ≥ 6 L/minute, except for patients with home ventilation), requirement for catecholamines, or hemodialysis (except for patients with chronic kidney disease) as defined for the PROGRESS study (Ahnert et al. (2019)).

### Brief description of the Figures

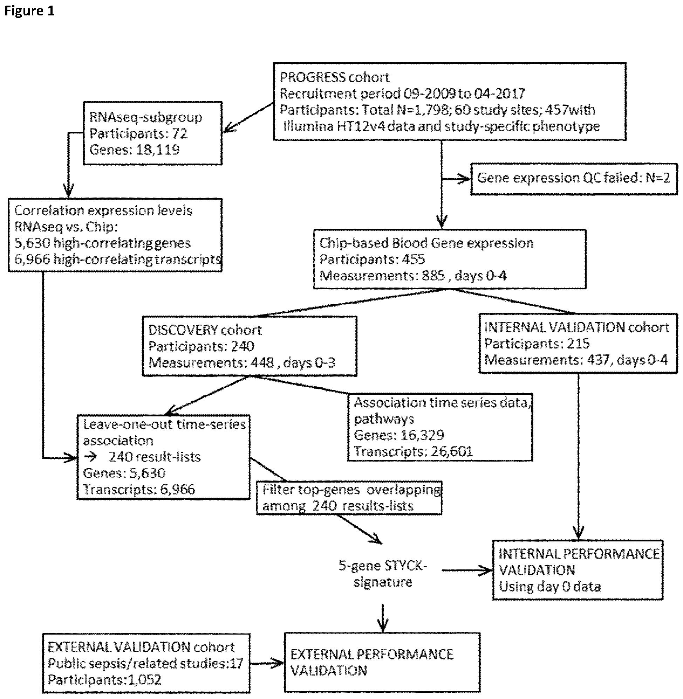

Figure 1: Flow chart and study design for the identification and validation of the 5-gene STYCK-signature. The Study of Progression of Community Acquired Pneumonia in the Hospital (PROGRESS) is a prospective multicenter observational cohort study that has been recruiting CAP patients requiring hospitalization. Comprehensive characterization regarding baseline characteristics and outcomes / scores of participants is shown in Table 2 and Table 3, respectively. Abbreviations: CAP: community-acquired pneumonia

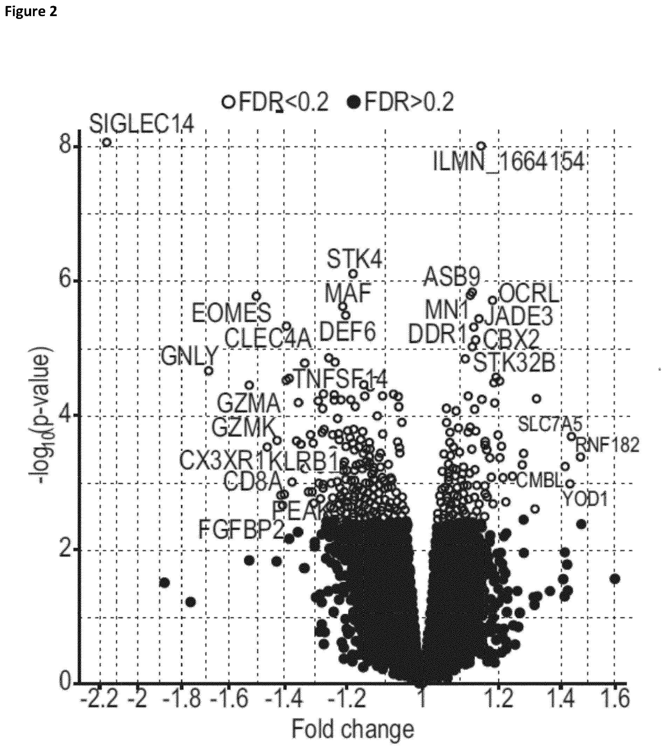

Figure 2: Whole-blood transcriptome in patients with CAP with cEP in the discovery cohort. Results of differential expression analysis shown as Volcano plot. Significantly altered gene expression (FDR ≤ 0.2) in patients reaching the cEP are marked as white points. Data were derived from the discovery cohort and analysis was adjusted for the SOFA score. Abbreviations: FDR: false discovery rate.

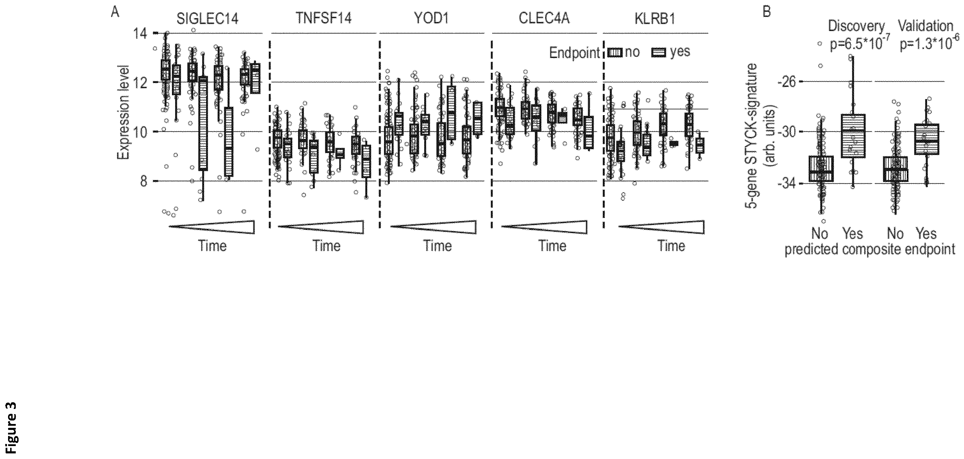

Figure 3: The 5-gene STYCK-signature and its individual genes are differentially expressed in patients with a severe disease course (A) Boxplot representation of microarray expression levels of the five genes of the STYCK-signature in cases (striped boxplots) or controls (non-striped boxplots). Time gradients correspond to the day of admission and the following three days at the hospital. (B) Combination of the five genes, in both, the discovery and internal validation cohort measured at the day of admission. P-values were calculated with Wilcoxon rank sum test. Abbreviation: arb. units: arbitrary units

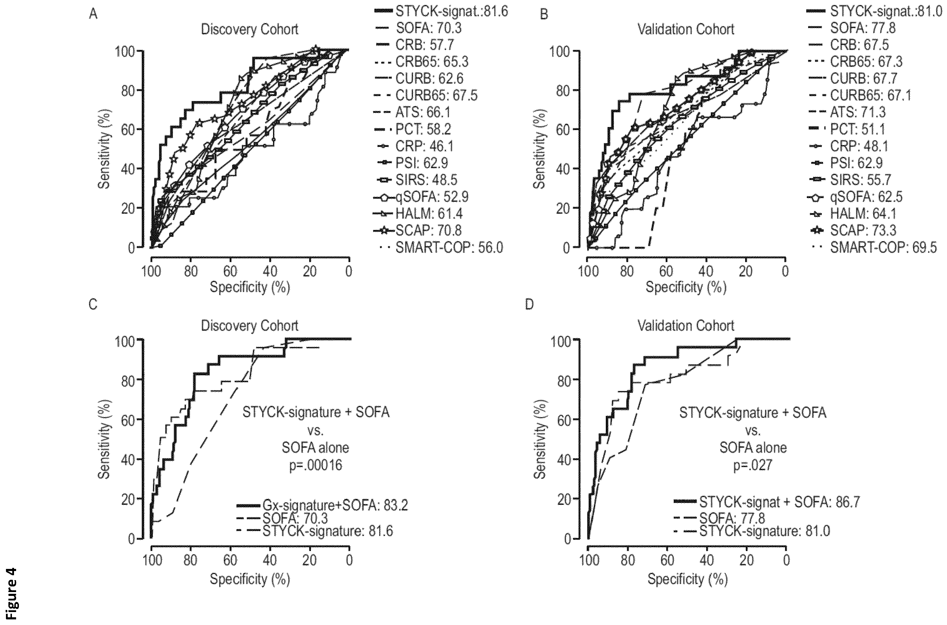

Figure 4: Prediction performance of the STYCK-signature is better than other proposed clinical scores. (A, B) Comparison of the performance of the STYCK-signature with clinical scores and CRP levels or Procalcitonin in (A) the discovery cohort and (B) the validation cohort. (C, D) Predictive performance of the combination of the STYCK-signature with the SOFA score in (C) the discovery cohort and (D) the validation cohort. Values in the legend are the area under the receiver operator characteristic curve, an AUC=0.5 corresponds to no predictive information. P-values were calculated using 2,000 bootstrap-samples. Abbreviations: ATS: American Thoracic Society minor criteria; AUC: area under the curve; CRB: similar as CURB without blood-urea nitrogen; CURB65, CRB65: as CURB, CRB but additionally considering age ≥65; CRP: C-Reactive Protein; CURB: Confusion, Urea, Respiratory rate, Blood pressure score; PCT: Procalcitonin level; PSI: Pneumonia Severity Index (Fine score); qSOFA: quick SOFA score; Halm: Halm score; SCAP: Severe CAP score; SIRS: Systemic Inflammatory Response System; SMART-COP: systolic blood pressure, multilobar chest radiography involvement, low albumin level, high respiratory rate, tachycardia, confusion, poor oxygenation, and low arterial pH score; SOFA: Sequential Organ Failure Assessment score

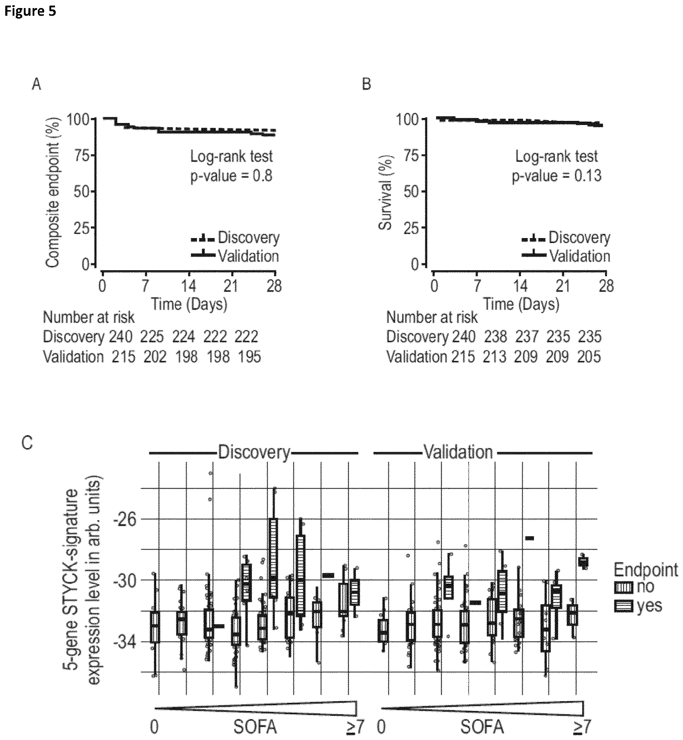

Figure 5: (A) cEP-free 28-day survival and (B) 28-day survival of patients from the discovery and validation cohorts. (C) Predictive performance of the signature for patients stratified by present disease severity (SOFA) at the time of admission. Different levels of the STYCK-signature for cases reaching the endpoint later on compared with controls are also present for patients with initially lower SOFA-values. Abbreviations: arb. units: arbitrary units

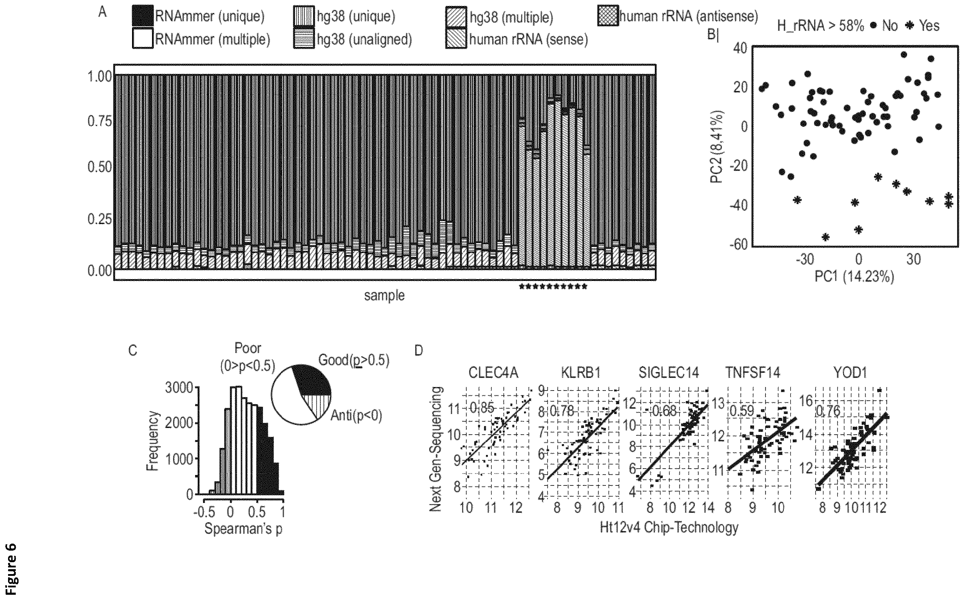

Figure 6: Correlation of Next Generation Sequencing (NGS) with HT12v4 Chip technology. (A,B) QC-Plots for next generation sequencing of the whole transcriptomes of 72 patients that were sequenced. A): To assess the sequencing library composition, each sample was subsampled to randomly 1 million trimmed paired-end reads. Subsampled reads were mapped using FastQ Screen in conjunction with bowtie2 iteratively against the RNAmmer database v1.2, human rRNA and the human genome assembly GRCh38/hg38. Human rRNA reads are divided into sense, which resembles endogenous rRNA and anti-sense RNA which indicate rRNA antisense probes from the rRNA depletion step. 10/72 Samples mapped with at least 55% against the human rRNA reference are depicted with asterisks. B) Principal components analysis of variance-stabilized counts based on the 5000 most variable genes (First and second principal components). Samples are displayed as asterisks, when corresponding subsampled reads mapped with at least 55% against the human rRNA reference. As they mapped in sense to rRNA transcripts, they resembled endogenous rRNA. The variation in the second component was partly explained by these 10 samples. Still, all NGS samples were included in the expression chip correlation analysis, as its aim was to identify transcripts that are robustly correlated, even in the presence of NGS-quality issues. (C) Correlation of transcript expression levels measured with HT12-v4 Chip Technology with expression levels measured with next-generation RNA sequencing (Illumina Inc.). This analysis included 22,453 out of 26,601 transcripts that could be matched to a gene from sequencing. Numbers shown are the correlation parameters rho, and the genes included in the signature are displayed as scatterplots. (D) Correlation of the expression levels of the five transcripts from the STYCK-signature measured with HT12-v4 Chip Technology (shown on the log-scale) with expression levels measured with next-generation RNA sequencing (shown as log-transformed counts) Abbreviations: hg38: human genome built 38; PC: principal component

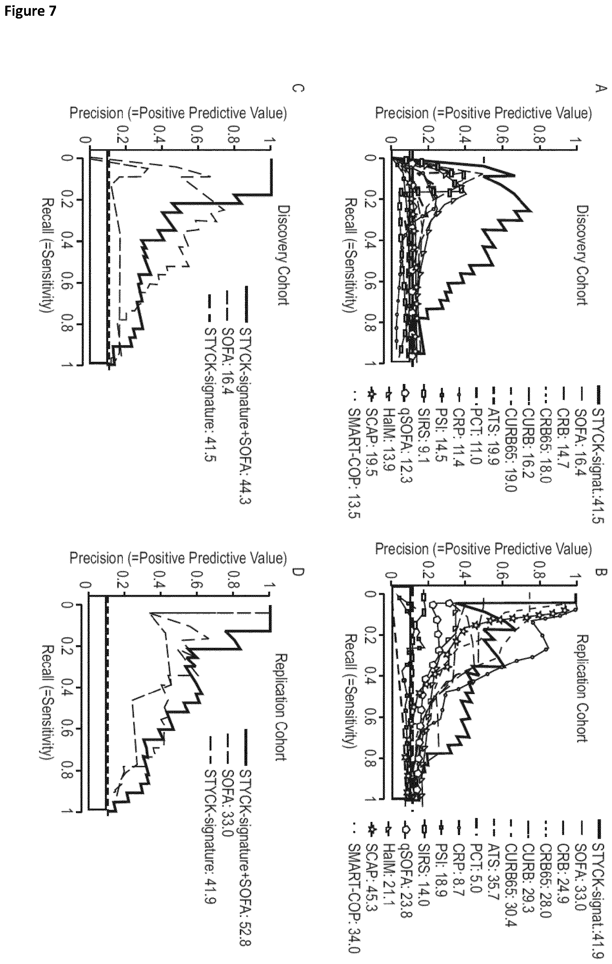

Figure 7: Predictive performance of the STYCK-signature in precision recall curves. (A, B) Comparison to clinical scores and serological markers in (A) the discovery cohort and (B) the validation cohort. Details on clinical scores are reported in Schubert et al. (2016). (C, D) Predictive performance in combining the STYCK-signature with the SOFA score in (C) the discovery cohort and (D) the validation cohort. Values are the area under the precision-recall curves. P-values were calculated using 2000 bootstrap-samples. Abbreviations: SOFA: Sequential Organ Failure Assessment.

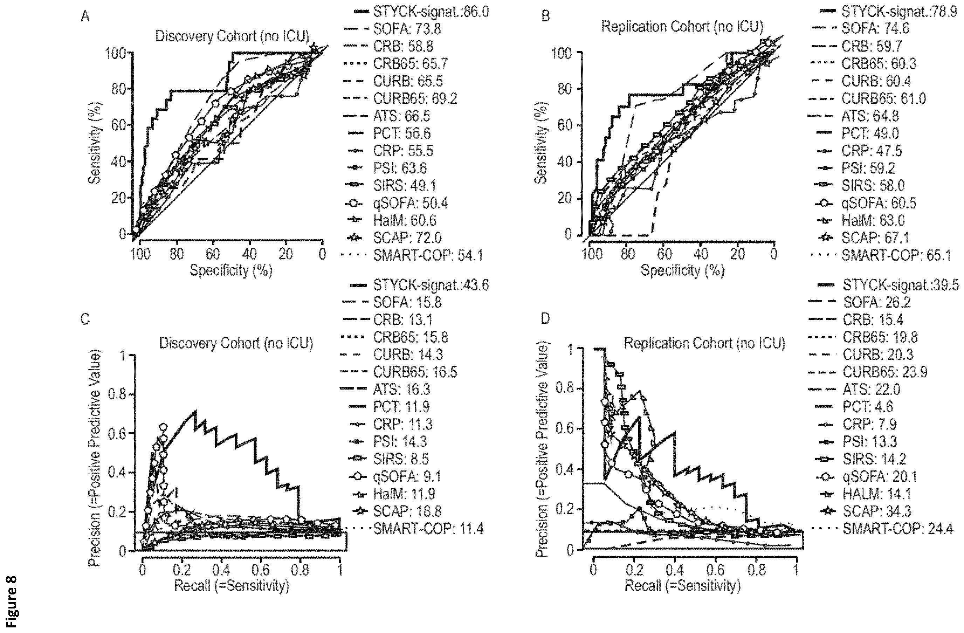

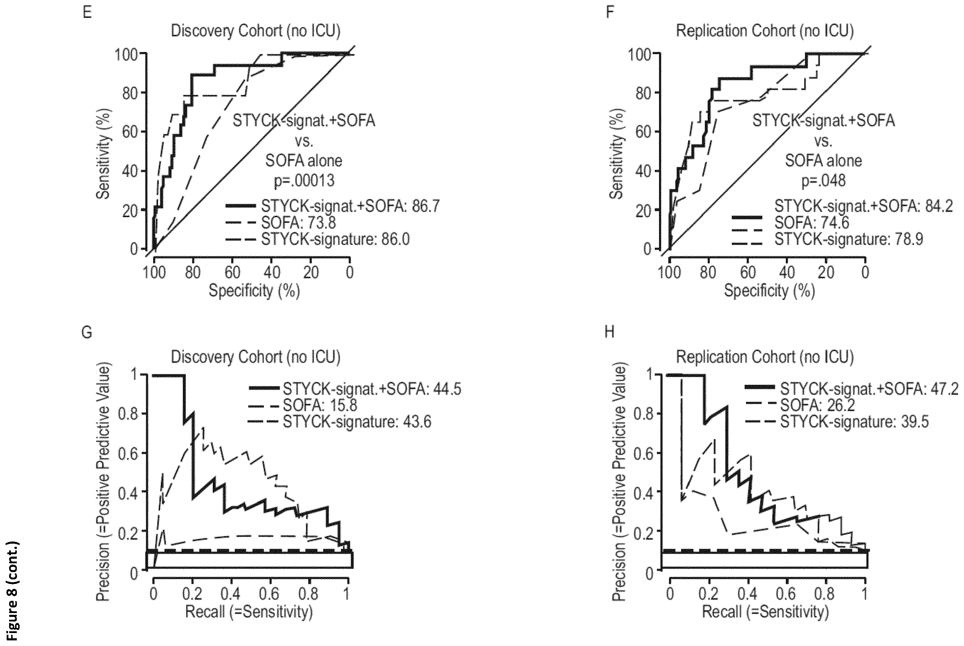

Figure 8: Predictive performance of the STYCK-signature is also better than other scores when excluding patients at ICU not fulfilling our criteria of qualified ICU. (A, B): Comparison of prediction performances of the STYCK-signature to 16 clinical scores quantified as AUC of the ROC curve in (A) the discovery cohort and (B) the validation cohort. Details on the clinical scores are provided in Schubert et al. (2016). (C, D): Same as (A, B), but prediction performance quantified as area under the precision-recall curves. (E, F): Predictive performance quantified as AUC of the ROC curve when combining the STYCK-signature with the SOFA score in (E) the discovery cohort and (F) the validation cohort. (G, H): Same as (E, F), but prediction performance quantified as area under the precision-recall curves. All values in the legend represent area under the curve, either of the ROC or of the precision-recall curve. P-values were calculated using 2000 bootstrap-samples. Abbreviations: SOFA: Sequential Organ Failure Assessment.

### Detailed description of the invention

The expression of genes as RNA molecules represents a fundamental biological functional and regulatory level. The amount of RNA produced by the cell is determined both by individual genetics and by environmental influences (e.g. infections). For processes of immune response and disease progression with systemic relevance, peripheral blood represents a highly relevant tissue, which is also easily accessible. The amount of specific RNA molecules found in the blood can be both diagnostically and prognostically relevant. Gene expression information from multiple genes can be combined to produce a score. In the present invention, it was possible to identify 5 probes from 47,231 measured gene probes and combine them into a predictive score with predictive power for a later severe course of CAP.

The invention describes a method of prognosis, prediction or risk stratification for mortality, disease outcome or ICU admission of a patient who has been diagnosed with community acquired pneumonia (CAP). This method comprises analysing a sample taken from a patient and specifically determining the expression level of a biomarker or a selected set of combined of biomarkers in said patient sample. The result is then correlated to a threshold value and in the case where it is above that threshold value, said patient is designated as at risk for severe disease course of CAP with likely ICU treatment and possible mortality.

The biomarker genes of the invention which are identified as predictive for prognosis and risk stratification of CAP patients are listed below in table 1. These biomarkers form the gene signature according to the invention. The biomarkers of the invention are detected as mRNA transcripts from blood samples of a patient upon admission to the hospital. Table 7 lists the known transcript variants of each biomarker, which are included into this application as SEQ ID NO: 1-13. Table 7 further lists the probe sequences which were used for detection of the transcripts (SEQ ID NO: 14-18).The probe sequences listed in table 7 are specific probes of the applied detection platform, however these sequences are only exemplary and not limiting. As the person skilled in the art is aware there are usually numerous possible design options available to obtain specific and reliable primers and probes for a sufficiently long target sequence. Any primer or probe sequence which specifically detects the biomarkers of SEQ ID NO: 1-13 is part of the present invention. Primers and probes are available and the skilled person in the art in aware on how to design and validate these. Thus, table 7 does not represent a comprehensive list of all possible probes for detection of the biomarkers of the invention.

**Table 1: Selected Biomarkers of the STYCK signature of the invention**

| **HGNC Symbol (Entrez Gene ID)** | **Full name and Aliases** |
|---|---|
| CLEC4A (#50856) | **C-type lectin domain family 4 member A:** |
| | DCIR, LLIR, CD367, DDB27, hDCIR, CLECSF6, HDCGC13P |
| KLRB1 (#3820) | **Killer cell lectin-like receptor B1:** |
| | CD161, CLEC5B, NKR, NKR-P1, NKR-P1A, hNKR- P1A |
| SIGLEC14 (#100049587) | **Sialic Acid Binding Ig-Like Lectin 14:** |
| | LLNLR-470E3.1 |
| TNFSF14 (#8740) | **Tumor necrosis factor superfamily member 14:** |
| | CD258, HVEML, LIGHT, LTg, TR2, TNLG1D |
| YOD1 (#55432) | **YOD1 Deubiquitinase:** |
| | DUBA8, OTUD2, OTU1, HIV-1- Induced Protease 7, DUBA-8, HsHIN7, PRO0907 |

| | |
|---|---|
| Abbreviations: HGNC: Human Genome Organization Gene Nomenclature Committee | |

In one aspect the present invention refers to a diagnostic method for the ex-vivo prognosis, prediction or risk stratification for mortality, disease outcome or ICU admission of a patient who has been diagnosed with community acquired pneumonia (CAP) comprising the steps of
a) providing a sample from the patient,
b) analysing the expression level of at least one nucleic acid in the sample,
   wherein the patient's prognosis, prediction or risk stratification is determined according to the expression levels of the at least one nucleic acid,
   wherein the at least one nucleic acid is selected from the group comprising CLEC4A, KLRB1, SIGLEC14, TNFSF14 and YOD1.

Starting point of the method is peripheral whole blood, from which with an appropriate method the expression levels (i.e. RNA quantity) of the genes according to table 1 are determined: *TNFSF14, YOD1, KLRB1, CLEC4A,* and *SIGLEC14.* In a preferred embodiment, the score is calculated from the respective log2-transformed expression values of the five genes. Following the procedure of Sweeney et al. 2015 and 2016, the geometric mean is first calculated for each patient from the four down-regulated genes *TNFSF14, KLRB1, CLEC4A* and *SIGLEC14* and multiplied by the weighting factor four according to the number of genes entered. The gene expression score used is then obtained by subtracting this value from the expression value of the upregulated gene *YOD1.*

A blood-based measurement of gene expression patterns, in contrast to the use of clinical scores, allows a faster, less subjective and also, from the patient's point of view, less burdensome survey, which can also be performed during the course of admission. The method of the invention relies on taking a blood sample which would be part of usual admission procedures anyway and therefore the assay of the invention does not present an additional inconvenience to the patient. The performance of the gene expression signature prediction is better than that of established clinical scoring systems.

In one embodiment of the invention the sample is selected from the group comprising blood, whole blood, blood serum, blood plasma, buffy coat, urine, sputum, tracheal secretions, cheek swabs and tissue samples and including other bodily fluids and secretions. Preferably, the sample is blood, whole blood or buffy coat. blood sample, most preferably a serum sample or a plasma sample. The samples may or may not be treated to stabilize and preserve RNA. Preferably, the sample is obtained from the patient as early as possible. Ideally the sample is taken upon admission of the patient to ensure transfer of the patient to the ICU as soon as possible should the prognosis indicate the need for ICU treatment.

In one embodiment of the invention at least one nucleic acid is selected from the group comprising CLEC4A, KLRB1, SIGLEC14, TNFSF14 and YOD1. These nucleic acids represent the biomarkers which are identified in the present invention as having significant predictive value for prognosis of CAP. Each of these biomarkers have predictive value on their own as presented in table 5.

However, combining the biomarkers of the invention into a combined predictive score as defined above further improves the predictive value of the method of the invention as shown in Figures 4, 7 and 8.

In further embodiments of the invention the method of invention involves expression level analysis of at least two, preferably at least 3, more preferably at least 4 and most preferably at least 5 of the nucleic acid biomarkers.

The predictive performance for limited selections of the biomarkers of the invention were calculated and are shown in table 6. Embodiments with a limited selection of biomarkers are predictive and can be used for the method of the invention. More limited subsets may represent useful embodiments to reduce costs and effort required to perform the prognostic test.

The dataset of table 6 further indicates the presence of a core subset consisting of biomarkers CLEC4A, KLRB1 and YOD1, preferably embodiments of the method of the invention comprise testing of at least two biosmarkers of the core subset or even more preferably all three biomarkers of the core subset. Embodiments of the invention which comprise the core subset have higher predictive values than alternative embodiments which do not or only partially comprise the core subset.

The biomarkers of the invention correspond to SEQ ID NO: 1-13 of the sequence listing. Hence, in one embodiment of the invention the analysed nucleic acids are selected from the group comprising SEQ ID NO: 1-13.

The method of the invention involves determining mRNA expression levels of the biomarkers of the invention. The person skilled in the art is aware of different available techniques which are useful for determining expression levels. Any possible procedure to reliably determine mRNA expression levels known to the skilled person is applicable for the method of the invention. Hence, analysis of the mRNA expression levels is performed according to a method selected from qPCR, qRT-PCR or digital PCR which involve fluorescence measurement of a fluorescently-labelled primer, fluorescently-labelled probe or a fluorescent DNA dye as detection agent. Further, sequencing or NGS methods, e. g., lonTorrent, and Microarrays may also be used to determine mRNA expression levels. Alternatively, mRNA expression levels of the gene signature may also be determined using mRNA-Arrays, gene chips or RNA-microarrays. In certain embodiments microfluidic devices which are capable of performing PCR or sequencing reactions may be used. Preferably, mRNA expression levels are determined by qRT-PCR. The advantage of both sequencing and PCR amplification approaches is that they can be performed automatically in high-throughput systems which reduces time and labor effort and thus achieves cost-efficient clinical tests, which can be used as standard tests.

In one embodiment of the invention the expression levels are normalized to general reference genes and or specific reference genes validated for blood samples. Normalization of expression levels serves to improve inter sample comparability by reducing the variability of the determined values. Hence, the predictive score of the method of the invention

In one embodiment of the invention the method of the invention provides a prediction for the likelihood of a composite endpoint consisting of 28-day mortality or qualified ICU admission with CAP-specific treatment. For the method of the invention a composite endpoint was chosen to cover both relevant possible endpoints in case of a severe disease course, i.e., 28-day mortality or qualified ICU admission. The gene signature of the invention is selected to provide a reliable prediction of the composite endpoint of the method.

The method of the invention as definded and described above achieves superior predictive power for prognosis and risk stratification of CAP patients. Superior performance of the method of the invention can be seen in Figure 4, wherein sensitivity versus specificity of the method of the invention is shown in comparison to alternative clinical scores. The method of the invention achieves an AUC of above 80. Accordingly, in one embodiment the method of the invention achieves an AUC of at least 65, preferably at least 70, more preferably at least 75, even more preferably at least 80 and most preferably at least 85.

Alternatively, the predictive performance of the method can be represented as shown in Figure 7 by presenting precision versus recall of the method of the invention. In this embodiment of the invention the AUC-PR of precision recall curves of the method of the invention is at least 20, preferably at least 25, preferably at least 30, more preferably at least 35, even more preferably at least 40 and most preferably at least 45.

Accordingly, in one embodiment the method of invention further comprises determination of a predictive score, wherein this predictive score refers to the likelihood of a composite endpoint consisting of 28-day mortality or qualified ICU admission. The predictive score which is provided by the method of the invention can be compared to a standard reference and based on the comparison a risk stratification of the patient or prognosis for the patient's disease course is obtained. The predictive score encompasses the likelihood of the patient for 28-day mortality or qualified ICU admission into a combined prediction. The prognosis for the patient is reached by comparing the predictive score to a reference or threshold value, wherein 28-day mortality or qualified ICU admission is predicted if the predictive score is above or below the reference or threshold value.

In a further embodiment of the invention the predictive score of the invention can be combined with established clinical scores. The combination of the predictive score of the invention which is based on the gene signature of the invention with another clinical score further improves the predictive values of the method of the invention. Preferably, the clinical score is selected from the groups comprising SOFA: Sequential Organ Failure Assessment Score; Variations of CURB criteria scores (CRB, CRB65, CURB, CURB65); ATS: Infectious Diseases Society of America/American Thoracic Society minor criteria for CAP severity; PCT: Procalcitonin assay; CRP: C-reactive protein; PSI: Pneumonia severity index; SIRS: Systemic inflammatory response syndrome criteria; qSOFA: quick SOFA (Sequential Organ Failure Assessment); Halm: Halm's criteria; SCAP: Severe Community-Acquired Pneumonia Score; SMART-COP Score (systolic blood pressure, multilobar infiltrates, albumin, respiratory rate, tachycardia, confusion, oxygen, and pH). Most preferably the clinical score is SOFA. Figures 7 and 8 present further possible improvement of the predictive value of the method of the invention when the score based on the gene signature of the invention is combinded with the SOFA score. The combinded test of the SOFA score and the score of the gene signature of the invention achieves superior results than each of the tests on their own.

For best outcome the method of the invention should be performed as early as possible, to ensure that patients who are expected to develop a severe disease according to the method of the invention are transferred to the ICU early to ensure adequate treatment. Hence, in one embodiment of the invention the method is performed on admission of the patient or shortly thereafter. The method of the invention may be performed at a later point as well, however since for patients who develop a severe disease benefit significantly from an early transfer to the ICU, an early prognosis is most preferred.

Another aspect of the invention is a nucleic acid that hybridizes under stringent conditions to one of the nucleic acids according to SEQ ID NO: 1-13, or the reverse complement thereof or any part thereof, or a nucleic acid that shares preferably at least 85%, 90%, 95% or 99% sequence identity with one of the sequences of SEQ: 1-13, or the reverse complement thereof or any part thereof. For determining mRNA expressions levels of the biomarker genes of the gene signature of the invention complementary probes or primers are required in accordance with the method of quantification. Hence, any suitable nucleic acid which may be used for specific and reliable detection and quantification of the expression levels of the gene signature are part of the present invention. Preferred embodiments of the nucleic acids are listed in table 7.

The nucleic acid according to the present invention is about 10 to 100 nucleotides in length.

To facilitate detection of the nucleic acid of the invention, the nucleic acid may comprise a detectable label. Preferably, the detectable label is a suitable fluorescent label for detection in the method of the invention. Any fluorescent or other label which can be detected within ,e. g., qPCR, sequencing or microarray techniques may be used. Under definitions a list of selected available fluorescent dyes is provided.

Another aspect of the present invention refers to the use of the nucleic acid according to the method of the invention for the prognosis, prediction or risk stratification for mortality, disease outcome or ICU admission of a patient who has been diagnosed with community acquired pneumonia (CAP).

Another aspect of the invention refers to a diagnostic kit for the prognosis, prediction or risk stratification for mortality, disease outcome or ICU admission of a patient who has been diagnosed with community acquired pneumonia (CAP), wherein the kit comprises:
a) a nucleic acid according to SEQ ID NO: 1-13;
b) reagents for nucleic acid amplification and/or quantification and/or detection.
c) optionally standards and/or internal standards and
d) reference data for patients under reference conditions,
e) a manual.

The kit of the invention is diagnostic kit for a quick bedside examination of the patient with CAP for direct risk stratification and prognosis of the patient upon hospital admission. The components of the kit are optimized for automation of the assay.

### Examples

### Example 1: Study design and participants

The Study of Progression of Community Acquired Pneumonia in the Hospital (PROGRESS) is a prospective multicenter observational cohort study that has been recruiting CAP patients requiring hospitalization in Germany and Austria (ClinicalTrials.gov: NCT02782013) (Ahnert et al. (2016)).

In a case-control design, the inventors compared patients without and with a severe disease course, defined as composite endpoint (cEP) including i.) 28-day all-cause mortality or ii.) qualified ICU admission defined as ICU with substantial respiratory support, requirement for catecholamines, or dialysis (Ahnert et al. (2019)). The study population was randomly separated into a discovery and validation cohort (see Figure 1 for a study flow chart). The protocol was approved by the ethics committee of the University of Jena (2403-10/08) and by locally responsible ethics committees for each study site, in accordance with Good Clinical Practice guidelines (Committee for Human Medicinal Products (2020)) and the provisions of the Declaration of Helsinki (World Medical Association (2020)). Written informed consent was obtained from the patients or their legal representatives.

The overall aim of the PROGRESS study was to identify clinical and molecular genetic factors determining or predicting severe disease courses in a hypothesis-free manner. Patients older than 18 years with a diagnosis of CAP were considered for inclusion. CAP was defined as i.) pulmonary infiltrate detected by chest X-ray, and ii.) at least two of the following symptoms: fever, cough, purulent sputum, shortness of breath/need for respiratory support, or crackling /rales on auscultation, dullness to percussion, or bronchial breathing. Patients admitted to hospital within the previous 28 days or with immunosuppresson were excluded (see Ahnert et al. (2019) for a detailed list of exclusion criteria).

Disease course and therapy were closely monitored by daily assessments and long-term follow-up. Comorbidities were assessed using the modified Charlson scores. Whole blood samples for gene expression analysis were sampled between day 0 (day of enrolment, further on termed admission) and day 5. Further details on sample and data collection are previously described (Ahnert et al. (2019).

### Definition of a severe disease course

A severe disease course was defined based on a composite endpoint (cEP) consisting of i.) 28-day all-cause mortality or ii.) admission to the ICU initial non-CAP-related ICU admission for CAP-specific treatment. These criteria were defined as substantial respiratory support (ventilation, extracorporeal oxygenation, or oxygen supplementation ≥ 6 L/minute, except for patients with home ventilation), requirement for catecholamines, or hemodialysis dialysis (except for patients with chronic kidney disease) as defined earlier for the PROGRESS study (Ahnert et al. (2019)).

### Building of the discovery cohort and validation cohort

The study population was randomly separated into a discovery and validation cohorts. Randomization was stratified for cEP and missingness of the covariates required for regression analysis. It was ensured that both cohorts did not differ with regard to clinical characteristics by repeating probabilistic random sampling if one of the relevant clinical characteristics differed with nominal statistical significance including smoking years, duration of hospital admission, diabetes mellitus, elements of the comorbidity scores, requirement of ICU stay or ventilation at any time point, death, worst observed level of SOFA score, or of several clinical scores (Ahnert et al. (2019)), or serum levels of procalcitonin, C-reactive protein, thrombocytes, leukocytes and day of gene expression measurement. Patient characteristics were compared with the U-Test.

### Example 2: RNA sample preparation and analysis

### RNA quantification

RNA was isolated from venous blood, DNase digested, concentrated, quantified, and quality controlled. For array-based gene expression analysis, purified RNA was hybridized to Illumina HT-12v4 Expression-BeadChips (Illumina, San Diego, CA, USA). Low-quality samples were removed, and data was log2-transformed, quantile-normalized, batch-corrected and filtered for minimum expression levels, resulting in 26,601 transcripts representing 16,329 unique genes. The probe sequences for the detection of the selected biomarkers on Illumina HT-12v4 Expression-BeadChips are listed below in table 7 under SEQ ID NOs.: 14-18.

For sequencing-based expression quantification, libraries were prepared using globin-mRNA depleted RNA and sequencing was performed with HiSeq2500 sequencing (Illumina, San Diego, CA, USA), with an average sequencing depth of 100 million clusters per sample and 2x100b paired-end reads. Data analysis included demultiplexing, trimming, filtering, removal of low-quality bases, quantification at gene-level, and quality-assessment.

### RNA isolation and globin transcript depletion

For transcriptome analysis, whole venous blood was collected into PAXgene blood RNA tubes (Qiagen, Hilden, Germany) and stored at -80 °C until processing. A PAXgene miRNA kit (Qiagen) was used for RNA isolation. In brief, frozen whole blood was thawed and equilibrated at room temperature for 2 h. The samples were centrifuged at 4,000g, the supernatant was decanted, and the pellets were resuspended in 4 ml of water. Further purification was carried out according to the manufacturer's instructions using a QlAcube. After two steps of DNase digestion (TURBO DNA-free Kit, Ambion) and sample concentration (RNA Clean & Concentrator-5 Kit, Zymo Research), the extracted RNA was quantified using a Qubit RNA Kit and a DeNovix instrument (Biozym). or Nanodrop 2000c (Thermo Scientific). The quality of RNA was assessed with a Bioanalyzer 2100 instrument (Agilent Technologies, Santa Clara, California, USA) with the RNA 6000 Nano Kit. Finally, for samples later used in RNA sequencing, globin mRNA was removed (max. RNA input: 1 µg per sample) using a GLOBINclear^{™}-Human Kit (Ambion), and the concentration and quality of the RNA were determined as described above.

### Array-based gene expression quantification

Purified RNA was dissolved at a concentration of 50-300 ng/µl prior to probe synthesis. Samples were hybridized to Illumina HT-12 v4 Expression BeadChips (Illumina, San Diego, CA, USA), and hybridization was measured using an Illumina HiScan according to the manufacturer's suggestions. Raw data for all 47,231 gene-expression probes were extracted by Illumina GenomeStudio without additional background correction. The data were further processed within R (version 3.4.3). Expression values were log2-transformed and quantile-normalized to allow parametric analysis Du et al. (2008)). Batch effects of BeadChip expression were corrected using an empirical Bayes method (Johnson et al. (2007)). During preprocessing, gene-expression probes detected by Illumina GenomeStudio as being expressed in less than 5% of the samples were excluded, as were probes still found to be significantly associated with processing batches after Bonferroni correction. These filters resulted in 26,601 valid gene-expression probes corresponding to 16,329 unique genes included in this analysis. Three criteria were used to remove samples of low quality (Kirsten et al. (2015)). First, the number of detected gene-expression probes of a sample was required to be within ± 4 interquartile ranges (IQRs) from the median. Second, the Mahalanobis distance of several quality characteristics of each sample was used ("signal of biotin-control probes, signal of low-concentration control probes, signal of medium-concentration control probes, signal of mismatch control probes, signal of negative control probes and signal of perfect-match control probes") (Cohen et al. (2007)), this had to be within median + 4 x IQR. Third, Euclidean distances of expression values 6 had to be within 5 x IQR from the median.

### Library generation and sequencing

Library preparation and sequencing was performed using 100 ng of globin-depleted RNA using the ScriptSeqTM Complete Gold Kit (Human/Mouse/Rat according to the manufacturer's instructions which also included ribosomal RNA depletion. AmpureXP Beads (Beckman Coulter) were used for all purification steps. High-throughput sequencing of sequencing libraries was performed with HiSeq2500 sequencing (Illumina, San Diego, CA, USA), with an average sequencing depth of 100 million clusters per sample and 2x 100b paired-end reads.

### RNA sequencing gene expression quantification and expression quality filtering

To facilitate the multistep analysis of the RNA sequencing datasets, we applied the workflow manager UAP (Kämpf et al. (2007)).

### Primary and secondary RNA sequencing data analysis

Demultiplexing of Illumina raw files was performed with Illumina bcl2fastq software v2.19 (https://emea.support.illumina.com/sequencing/sequencing_software/bcl2fastq-conversion-software.html). Paired-end FASTQ reads were trimmed and filtered using AdaptorRemoval v2.2.1 (Schubert et al. (2016)) with additional parameters to trim ambiguous bases (N) at the 5'/3' termini (--trimns), remove low-quality bases (--trimqualities, --minquality 20) and keep reads with a minimum read length of 30 bp (--minlength 30). Transcript abundancy estimation of each sample was conducted using Kallisto v0.44.0 (Bray et al. (2016)) by specifying a forward-stranded library. The human transcriptome FASTA file was downloaded from GENCODE (release 28 GRCh38.p12) and used to create a Kallisto index. Gene-level quantifications were generated from the Kallisto-estimated counts per transcript using tximport v1.8.0 with default parameters (Soneson et al. (2015)).

### RNA sequencing quality control

For each sample, a subsample of 1 million trimmed paired-end reads was randomly chosen by fastq-sample v0.8 (https://homes.cs.washington.edu/^{~}dcjones/fastq-tools/) using default parameters. Sample quality control was determined using FastQC v0.11.5 (https:// www.bioinformatics.babraham.ac.uk/projects/fastqc/) to assess base call accuracy and Preseq v2.0.2 (Daley et al. (2013)) to evaluate library complexity. These subsamples were aligned to the human reference genome GRCh38/hg38 using HISAT2 v2.10 (Kim et al. (2019)). Duplication metrics were collected using the Picard tools v2.3.0 (http://broadinstitute.github.io/picard/) function MarkDuplicates using BAM files generated by HISAT2. Picard's CollectRnaSeqMetrics function was used to determine mapping percentages on intergenic, intronic, coding and UTR regions as well as gene body coverage. RSeQC v2.6.4 (Wang et al. (2012)) was applied to determine the read GC content, junction saturation, read pair inner distance, and strandness of reads. Aggregated data visualization for the secondary analysis and quality control was performed using the MultiQC (Ewels et al. (2016)) framework. FastQ Screen v0.11.1a (Wingett et al. (2018)) in conjunction with bowtie2 (Langmead et al. (2018)) was applied to assess RNA library composition. In addition, using an iterative approach, we calculated the fraction of reads mapped against human rRNA transcripts to assess rRNA depletion efficacy during RNA library preparation.

For principal component analysis (PCA), read counts were normalized using a variance-stabilizing transformation implemented in DESeq2 v1.20.0 (Love et al. (2014)); the option "blind = TRUE" was applied to compare samples in an unbiased manner. PCA of samples was performed based on the 5000 most variable genes.

All NGS samples were included in correlation analysis, as its aim was to identify transcripts that correlate robustly when comparing data from expression chips and NGS, even in the presence of NGS-quality issues.

### Gene expression and pathway association analysis

This study was a case-control design in which patients reaching the cEP were compared with patients that did not reach the cEP. Only expression data measured before reaching the cEP was analyzed. Gene expression association analysis was performed in the discovery cohort first, applying a mixed effects model as implemented in the R add-on package limma 3.38.3 (Ritchie et al. (2015)). Repeated measurements of gene expression were considered as random intercepts and we adjusted for covariates age, sex, BMI, smoking within the last 12 months, comorbidities, SOFA score, and relative proportion of neutrophils and lymphocytes. Missing percentages of neutrophils and lymphocytes among leukocytes were inferred using CIBERSORT (Newman et al. (2015)). The false discovery rate (FDR) and proportion of null values of all tested hypotheses (Eta 1) were calculated based on empirical null modeling and Grenander-density approaches, as implemented in the R package fdrtool1.2.15 (Strimmer et al. (2008)). For pathway enrichment and activation analysis, we used Ingenuity Pathway Analysis (Krämer et al. (2014)). When analyzing the correlation of effect sizes from two different association analyses, we quantified the overall similarity R2 as the explained variance in a linear regression model.

### Assessment of robustness of gene expression measurement

To enable a robust measurement of a gene expression signature in a future prognostic test, the measurement of gene expression should be independent of the RNA measurement technology used. Therefore, we excluded genes from our search for a predictive expression signature that were not reliably measurable in an independent sequencing technique. To analyse this issue, we used the data from RNA sequencing on the subset of 72 of the included participants. We correlated all gene expression levels from the expression chip data with expression levels from the RNA sequencing. We considered transcripts robustly measurable with both techniques when correlation between chip data and sequencing data showed a statistically significant rank-correlation ρ≥0.5 and FDR≤5% rate.

### Identification of the predictive signature

For signature identification, we used the discovery cohort (N=240) and performed cross-validation by creating 240 individual leave-one-out datasets using the robustly measurable transcripts, only. For each of the datasets, we performed mixed effects modelling association with the same time-series regression model to utilize multiple time points per individual. This resulted in 240 association lists. These lists were filtered to identify the most promising predictive transcripts using the following steps. a) Transcripts whose expression was below a minimum fold-change (FC) and above a minimal FDR were removed to create a top list for each model. Several cutoffs were tested for this purpose (fold-changes between 1.1-1.5 in steps of 0.01, maximum FDR of 0.05, 0.1, 0.2, or 0.5) as discussed here (Sweeney et al. (2017)) (we later identified a fold-change of 1.33 and an FDR of 0.2 as the optimum). b) Overexpressed genes that were actually expressed in less than 10% of all cases, and under expressed genes that were expressed in less than 10% of controls were filtered to remove genes with low expression level. c) Multiple transcripts from the same gene were filtered such that only unique genes remained in each top list. d) Genes highly correlated with the expression levels of other genes from the same top list (i.e. keeping only one of the genes when several genes had a Pearson-correlation higher than 0.8) were filtered, and we favored for step c and d transcripts with higher effect sizes, those not mapping at multiple locations in the human genome and higher expression levels to increase robustness. e) Genes that switched effect size direction upon including all other genes of the respective top list as covariates in association analysis, were filtered to favor genes with consistent effects.

Next, 240 candidate signatures were determined on the basis of the steps a)-e) as previously described (Sweeney et al. (2015), Sweeney et al. (2016)). Briefly, for each patient, the geometric mean of the log2-transformed expression levels of downregulated genes was subtracted from that of the upregulated genes. Thereby, the geometric means are weighted by the number of down- and upregulated genes.

For each combination of fold-change cutoffs and FDR cutoffs (step a)), the predictive performance to predict the cEP (quantified as the area under the curve (AUC) from the receiver operator characteristic (ROC) analysis) of the expression scores measured on the day of admission was analyzed for each of the 240 left-out patients. The resulting AUCs were used to rank the parameter combination of FC and FDR considered in step a).

To unite each of the 240 gene lists, we identified genes that frequently appeared across multiple top lists. We tested different cutoffs for multiple appearances, i.e., genes that were present in 50%, 60%, 70%, 80% and 90% of all 240 top lists, using finally the 90% cut-off value. Expression scores were calculated from overlapping genes, again using chip data measured from whole blood on the day of admission. The predictive performance of these scores was evaluated on the basis of the AUC of the complete discovery cohort. The signature with the optimal AUC was selected. Again, the resulting AUC was used for a second ranking of the FC and FDR parameters used at step a), eventually resulting in the final choice of these parameters.

### Evaluation of combination of the signature and the SOFA score

AUCs of ROCs were calculated with the R package pROC 1.18.0 (Robin et al. (2011)). Here, confidence intervals as well as comparisons of two ROC-curves were calculated using 2000 stratified bootstrap samples, p-values of single analyses were calculated based on the Wilcoxon-Mann-Whitney U-statistic. When combining two scores to analyze combined prediction, the unweighted sum of both scaled scores was utilized.

### Example 3: Experimental results of gene expression analysis

### Cohorts

In the current study, the PROGRESS cohort (Ahnert et al. (2019)) consisted of 1,798 hospitalized CAP patients from 60 study sites. Gene expression was measured in 1,196 whole blood samples of 545 patients, from which 10 expression-arrays were excluded for quality reasons. Overall, these filtered samples comprised 1,186 gene expression datasets, measured in 543 patients. After exclusion of patients immediately transferred to the ICU for CAP-specific reasons, this study cohort consisted of 455 patients with 885 utilizable gene expression data sets repeatedly measured between day 0 and day 4. Patients were classified into 49 cases and 406 controls depending on whether they reached later on the cEP or not, respectively. The composite endpoint operationalized severe course of CAP defined as qualified ICU admission and/or 28-day mortality (15 of the 49 cases died). Of note, this also included 43 CAP patients with ICU at admission not fulfilling the criteria for qualified ICU admission, with 10 of those 43 patients reaching the cEP later. All participants were retrospectively randomly divided into a discovery cohort (n = 240) and a validation cohort (n = 215, Figure 1 for a study flow chart and Figure 5A and B for Kaplan-Meier plots). The cohort's characteristics are provided in Tables 1 and 2.

**Table 2: Baseline characteristics of participants in the discovery and validation cohorts.**

| **Demographics** | **Discovery Cohort (n=240)** | **Validation Cohort (n=215)** | **p Value** | **Total (n=455)** | **Missing Values (n,%)** |
|---|---|---|---|---|---|
| **Age, years, median [IQR]** | 66.00 [49.00, 75.00] | 65.00 [45.00, 74.00] | 0.332 | 65.00 [47.50, 75.00] | 0 (0%) |
| **Sex (male), n (%)** | 153 (63.7) | 135 (62.8) | 0.909 | 288 (63.3) | 0 (0%) |
| **Sex (female), n (%)** | 87 (36.2) | 80 (37.2) | 0.909 | 167 (36.7) | 0 (0%) |
| **BMI** | 26.47 [23.09, 30.05] | 25.83 [22.46, 29.76] | 0.586 | 26.22 [22.64, 30.02] | 2 (0.4%) |
| **Smoking within last year (n (%))** | 82 (34.7) | 87 (40.7) | 0.232 | 169 (37.1) | 5 (1.1%) |
| **Smoking years, median [IQR]** | 12.50 [0.00, 30.00] | 16.50 [0.00, 30.00] | 0.568 | 15.00 [0.00, 30.00] | 51 (11.2%) |
| **Length of hospital stay** | 7.00 [5.00, 10.00] | 7.00 [5.00, 10.00] | 0.537 | 7.00 [5.00, 10.00] | 14 (3.1%) |
| **Comorbidities** | | | | | |
| **None** | 102 (42.5) | 91 (42.3) | 1.000 | 193 (42.4) | 0 (0%) |
| **Diabetes (n (%))** | 43 (17.9) | 45 (20.9) | 0.488 | 88 (19.3) | 0 (0%) |
| **CHD (n %))** | 81 (34.6) | 64 (30.3) | 0.389 | 145 (31.9) | 10 (2.2%) |
| **Chron. cerebral dis. (n (%))** | 13 (5.4) | 9 (4.2) | 0.712 | 22 (4.8) | 2 (0.4%) |
| **CKD (n (%))** | 22 (9.3) | 24 (11.4) | 0.567 | 46 (10.1) | 7 (1.5%) |
| **CLD (n (%))** | 3 (1.3) | 9 (4.2) | 0.099 | 12 (2.6) | 3 (0.7%) |
| **CRD (n (%))** | 83 (34.7) | 60 (28.2) | 0.163 | 143 (31.4) | 3 (0.7%) |
| **Comorbidity-score median [IQR]** | 1.00 [0.00, 2.00] | 1.00 [0.00, 2.00] | 0.894 | 1.00 [0.00, 2.00] | 0 (0%) |

| | | | | | |
|---|---|---|---|---|---|
| Abbreviations: CHD: chronic heart disease; CKD: chronic kidney disease; CLD: chronic liver disease; CRD: chronic respiratory disease; IQR: interquartile range. | | | | | |

**Table 3: Outcomes and scores of participants in the discovery and validation cohorts.**

| **Outcome/Scores** | **Discovery Cohort (n=240)** | **Validation Cohort (n=215)** | **p Value** | **Total (n=455)** | **Missing Values (n,%)** |
|---|---|---|---|---|---|
| **Composite EP (N (%))** | 25 (10.4) | 24 (11.2) | 0.916 | 49 (10.8) | 0 (0%) |
| **Future ventilation** | 17 (7.2) | 14 (6.6) | 0.94 | 0 | 8 (1.8%) |
| **28-day mortality (N (%))** | 5 (2.1) | 10 (4.7) | 0.205 | 15 (3.3) | 0 (0%) |
| **Worst future SOFA-score, median [IQR]** | 2.00 [2.00, 3.00] | 2.00 [2.00,4.00] | 0.665 | 2.00 [2.00, 4.00] | 45 (9.9%) |
| **CRB65, median [IQR]** | 1.00 [1.00, 2.00] | 1.00 [1.00, 2.00] | 0.881 | 1.00 [1.00, 2.00] | 0 (0%) |
| **CURB65, median [IQR]** | 2.00 [1.00, 2.00] | 2.00 [1.00, 3.00] | 0.986 | 2.00 [1.00, 2.00] | 0 (0%) |
| **ATS minor crit., median [IQR]** | 2.00 [1.00, 3.00] | 2.00 [1.00, 3.00] | 0.747 | 2.00 [1.00, 3.00] | 0 (0%) |
| **ATS minor crit. >2 (N (%))** | 86 (35.8) | 76 (35.3) | 0.992 | 162 (35.6) | 0 (0%) |
| **PSI, median [IQR]** | 3.00 [2.00, 4.00] | 3.00 [2.00, 4.00] | 0.737 | 3.00 [2.00, 4.00] | 0 (0%) |

| **Outcome/Scores** | **Discovery Cohort (n=240)** | **Validation Cohort (n=215)** | **pVaue** | **Total (n=455)** | **Missing Values (n,%)** |
|---|---|---|---|---|---|
| **SIRS median [IQR]** | 2.00 [2.00, 2.00] | 2.00 [2.00, 2.00] | 0.292 | 2.00 [2.00, 2.00] | 0 (0%) |
| **quick SOFA, median [IQR]** | 1.00 [0.00, 1.00] | 1.00 [0.00, 1.00] | 0.58 | 1.00 [0.00, 1.00] | 0 (0%) |
| **Halm, median [IQR]** | 2.00 [1.00, 3.00] | 2.00 [1.00, 3.00] | 0.804 | 2.00 [1.00, 3.00] | 0 (0%) |
| **SCAP, median [IQR]** | 7.50 [5.00, 15.00] | 6.00 [0.00, 15.00] | 0.355 | 6.00 [0.00, 15.00] | 0 (0%) |
| **smartCOP, median [IQR]** | 3.00 [2.00, 4.00] | 3.00 [1.00, 4.00] | 0.731 | 3.00 [1.00, 4.00] | 0 (0%) |
| **PCT, median [IQR]** | 0.34 [0.10, 3.18] | 0.22 [0.12, 1.85] | 0.196 | 0.26 [0.11, 2.48] | 76 (16.7%) |
| **CRP, median [IQR]** | 168.95 [96.05, 244.65] | 158.90 [90.80, 229.70] | 0.503 | 165.20 [94.25, 241.90] | 28 (6.2%) |
| **Intensive care (n (%))** | 22 (9.2) | 23 (10.7) | 0.697 | 45 (9.9) | 0 (0%) |
| **Thrombocytes, cells/µL, median [IQR]** | 221500.00 [168750.00, 288750.00] | 232000.00 [166000.00, 293000.00] | 0.514 | 226000.00 [167000.00, 292000.00] | 26 (5.7%) |
| **Leukocytes, cells/µL, median [IQR]** | 11700.00 [9160.00, 17300.00] | 11700.00 [8900.00, 15982.50] | 0.482 | 11700.00 [8920.00, 16400.00] | 14 (3.1%) |
| **Lymphocytes, %, median [IQR]** | 10.10 [5.95, 15.25] | 9.22 [6.00, 15.21] | 0.968 | 9.83 [5.96, 15.29] | 187 (41.1%) |
| **Total neutrophils, %, median [IQR]** | 81.65 [72.70,88.00] | 82.20 [73.54, 87.40] | 0.751 | 81.72 [73.05, 88.00] | 185 (40.7%) |

| | | | | | |
|---|---|---|---|---|---|
| Abbreviations: ATS minor crit: American Thoracic Society minor criteria; EP: endpoint; IQR: interquartile range; PCT: procalcitonin; PSI: pneumonia severity index, SCAP: Severe Community Acquired Pneumonia score; SOFA: Sequential Organ Failure Assessment score; CRP: C-reactive protein; SIRS: systemic inflammatory response syndrome. | | | | | |

### Gene expression is strongly associated with future disease course in patients reaching cEP

First, the inventors performed association analysis of whole-blood gene expression time-series data of 16,329 genes in the discovery cohort, comparing patients reaching the cEP as cases vs. those who did not. Note that only expression data of cases before reaching the cEP were used. This revealed that about 11% of the transcriptome was differentially expressed, with 52 and 349 genes differentially expressed at 5% and 20% FDR, respectively. The majority of these genes had reduced expression in cases (see Figure 2A for a volcano-plot, Table 4 for summarized associations). Gene-based effect sizes remained almost the same when adjusting for SOFA, supporting a largely SOFA-independent contribution of gene expression to the prediction of severe disease course.

**Table 4: Association between cEP status and gene expression levels in the discovery cohort.**

| **Case/Control (with cEP/ without cEP)** | **Adjusted for SOFA at admission** | **Eta1 (% Transcriptome Affected)** | **Expression Change Direction** | **Min. p Value** | **Number of significant Genes (FDR 5%/20%)** |
|---|---|---|---|---|---|
| **23/210 patients, 39/401 measurements** | Yes | 11% | Increased | 9.35*10-9 | 18/134 |
| | | | Decreased | 8.28*10⁻⁹ | 34 / 215 |
| | No | 12% | Increased | 1.13*10⁻⁹ | 43/210 |
| | | | Decreased | 4.48*10⁻⁸ | 105/416 |

| | | | | | |
|---|---|---|---|---|---|
| Results are shown with and without adjustment on the current SOFA score. The number of measurements is larger than the number of patients as analysis included repeated measurements, if available. Abbreviations: cEP: composite endpoint (CAP-specific treatment in the ICU or 28-day mortalty); FDR: false discovery rate. | | | | | |

### A 5-gene-signature ("STYCK") predicts severe course of disease in the discovery and validation cohort

Next, the inventors aimed to identify a gene expression signature predictive for severe course of CAP using the discovery cohort. To increase the independence of the signature regarding measurement technology, only genes were considered, whose expression was significantly correlated (ρ≥0.5, FDR≤0.05) when measuring a subset of 72 samples with a different technology (Illumina RNA sequencing, see Figure 6A and B for quality control plots). This holds for 6,966 transcripts (26% of all included transcripts, Figure 6C). Furthermore, a certain minimum absolute fold-change of 1.1 was required, in addition to significance (FDR≤0.5), expression level (expressed in ≥10% of cases or controls), robust uni- and multivariate associations (i.e. same effect size direction in uni- and multivariate models) and good performance (i.e. high AUC-rank in predicting the cEP with expression data from admission in a leave-one-out prediction scheme, see Example 2 for details).

The authores identified a 5-gene-signature meeting these criteria, including one gene upregulated in cases (YOD1, involved in endoplasmic reticulum stress response) and four genes downregulated in cases (SIGLEC14, CLEC4A, and KLRB1, all encoding lectin carbohydrate-binding proteins bound to immune cells; TNFSF14, a member of the TNF-ligand family) termed "STYCK". Differential expression on gene level is shown in Figure 3A and Table 5, and differential expression of the signature in Figure 3B. The prediction of the cEP by this 5-gene-signature had an AUC of 0.82 (95% Cl 0.71-0.91, p=6.5*10-7) and considerably higher than that of the individual five genes (AUC 0.61 to 0.73, Table 5). This was further supported when investigating clustering of the expression levels of the five genes in all patients, as no strong subclusters were observed. Measurability of these genes using RNAseq was good (ρ in between 0.59-0.85, Figure 6D).

Each of these genes and the STYCK-signature itself was associated with severe disease course in the independent validation cohort at least at nominal significance level (Table 5 for individual genes and Figure 3B for the signature). The AUC in the validation cohort was 0.81 (95%CI 0.70-0.91, p=1.3*10-6), therefore providing robustness to the STYCK-signature.

**Table 5: Association of signature genes in the discovery and validation cohort.**

| **Cohort (Cases/Controls)** | **HGNC** | **FC (95% Cl)** | **p Value** | **FDR** | **AUC (95% CI)** |
|---|---|---|---|---|---|
| **Discovery (23/210 patients, 39/401 measurements)** | TNFSF14 | -1.33 (-1.51; -1.17) | 1.64*10⁻⁵ | 0.0195 | 0.64 (0.51; 0.77) |
| | YOD1 | 1.44 (1.16; 1.78) | 1.01*10⁻³ | 0.119 | 0.73 (0.62; 0.83) |
| | KLRB1 | -1.36 (-1.60; -1.15) | 2.41*10⁻⁴ | 0.0641 | 0.69 (0.56; 0.81) |
| | CLEC4A | -1.39 (-1.60; -1.21) | 4.50*10⁻⁶ | 8.44*10⁻³ | 0.72 (0.59; 0.85) |
| | SIGLEC14 | -2.16 (-2.79; -1.67) | 8.27*10⁻⁹ | 1.11*10⁻⁴ | 0.61 (0.47; 0.74) |
| **Validation (21/191 patients, 39/394 measurements)** | TNFSF14 | -1.18 (-1.34; -1.04) | 9.50*10⁻³ | 0.012 | 0.56 (0.42; 0.69) |
| | YOD1 | 1.45 (1.17; 1.78) | 5.32*10⁻⁴ | 0.0013 | 0.66 (0.52; 0.79) |
| | KLRB1 | -1.21 (-1.43; -1.03) | 1.96*10⁻² | 0.02 | 0.68 (0.56; 0.79) |
| | CLEC4A | -1.28 (-1.47; -1.11) | 5.27*10⁻⁴ | 0.0013 | 0.65 (0.52; 0.77) |
| | SIGLEC14 | -1.52 (-1.95; -1.19) | 1.00*10⁻³ | 0.0017 | 0.66 (0.55; 0.76) |

| | | | | | |
|---|---|---|---|---|---|
| Regression model of the association of each gene adjusted on age, sex, comorbidity, cell counts, smoking, SOFA-score and body mass index.. AUC quantifies prediction accuracy of cEP using expression data from day of admission, only. The FDR accounts for multiple testing of all genes of the discovery panel and the 5 tested genes in the validation panel. Abbreviations: AUC: area under the curve; Cl: confidence interval; FC: fold change; FDR: false discovery rate; HGNC: Human Genome Organisation Gene Nomenclature Committee (https://www.genenames.org). | | | | | |

### The predictive performance of the signature outperforms other clinical scores

The inventors then compared the predictive performance of the signature with 14 clinical markers and scores. Details on these scores have been previously reported (Ahnert et al. (2019)). Only data from the day of admission were used (23/208 and 23/182 cases/controls for the discovery and validation, respectively). As shown in Figure 4A and B, the STYCK signature had the best predictive performance considering the AUC under the ROC in the discovery and validation cohort. Furthermore, the ability to predict severe disease outcome was significantly improved when the SOFA score and the STYCK-signature were combined (referred to as SOFA+) in contrast to SOFA alone (p=0.00016 and p=0.027 for the discovery and validation cohorts, respectively, Figure 4C and D). This was supported by heightened levels of the 5-gene-PROGRESS-signature in cases also with low SOFA at the time of the prediction (Figure 5C).

Second, the inventors quantified predictive performance as the area under the precision-recall curve. Again, better performance of the STYCK signature relative to most of the 14 clinical scores in both cohorts (Figure 7) were found. These results were also confirmed in a sensitivity analysis when excluding CAP patients at ICU not fulfilling the criteria for qualified ICU admission (Figure 8).

The robustness of the prognostic method using the selected biomarkers was tested by calculating the predictive performance for different subsets of the selected biomarkers. For each possible combinatorial subset consisting of 1, 2, 3, 4 or all 5 of the selected biomarkers the predicitve performance was determined. The results are listed in table 6. Both, AUC values of the ROC for prediction accuracy and AUC-PR for precision recall curves were calculated for each subset in both the discovery and validation cohorts each with and without inclusion of SOFA clinical score. The data show that the selected biomarkers of the invention provide stable and reliable predictions even in smaller subsets. Prediction performance improves for larger subsets, however embodiments with a limited selection of biomarkers are predictive and can be used. The dataset further indicates a core subset consisting of biomarkers CLEC4A, KLRB1 and YOD1, preferably embodiments of the method of the invention comprise testing of at least two biomarkers of the core subset or even more preferably all three biomarkers of the core subset.

**Table 6: Determining predictive performance for specific subsets of the biomarker set of the invention within the discovey and validation cohorts in combination with and without the SOFA clinical score. The AUC ROC and AUC-PR values are shown scaled to the range 0 and 1**

| **Prediction Accuracy (AUC ROC)** | | | **Discovery cohort** | | | **Validation cohort** | | |
|---|---|---|---|---|---|---|---|---|
| **N (Gene)** | **ID** | **Genes** | **AUC (genset)** | **AUC SOFA** | **Combined AUC** | **AUC (genset)** | **AUC SOFA** | **Combined AUC** |
| 5 | 5_V1 | CLEC4A, KLRB1, SIGLEC14, TNFSF14, YOD1 | 0,816 | 0,703 | 0,832 | 0,810 | 0,778 | 0,867 |
| 4 | 4_V5 | CLEC4A, KLRB1, SIGLEC14, YOD1 | 0,815 | 0,703 | 0,825 | 0,830 | 0,778 | 0,870 |
| 4 | 4_V4 | CLEC4A, KLRB1, SIGLEC14, TNFSF14 | 0,810 | 0,703 | 0,825 | 0,770 | 0,778 | 0,853 |
| 4 | 4_V3 | CLEC4A, SIGLEC14, TNFSF14, YOD1 | 0,755 | 0,703 | 0,810 | 0,756 | 0,778 | 0,843 |
| 4 | 4_V2 | KLRB1, SIGLEC14, TNFSF14, YOD1 | 0,778 | 0,703 | 0,806 | 0,804 | 0,778 | 0,860 |
| 4 | 4_V1 | CLEC4A, KLRB1, TNFSF14, YOD1 | 0,813 | 0,703 | 0,833 | 0,754 | 0,778 | 0,833 |
| 3 | 3_V10 | CLEC4A, KLRB1, SIGLEC14 | 0,794 | 0,703 | 0,812 | 0,784 | 0,778 | 0,855 |
| 3 | 3_V9 | CLEC4A, SIGLEC14, YOD1 | 0,769 | 0,703 | 0,812 | 0,784 | 0,778 | 0,855 |
| 3 | 3_V8 | KLRB1, SIGLEC14, YOD1 | 0,766 | 0,703 | 0,803 | 0,813 | 0,778 | 0,863 |
| 3 | 3_V7 | CLEC4A, KLRB1, YOD1 | 0,803 | 0,703 | 0,827 | 0,751 | 0,778 | 0,826 |
| 3 | 3_V6 | CLEC4A, SIGLEC14, TNFSF14 | 0,731 | 0,703 | 0,794 | 0,713 | 0,778 | 0,833 |
| 3 | 3_V5 | KLRB1, SIGLEC14, TNFSF14 | 0,762 | 0,703 | 0,800 | 0,760 | 0,778 | 0,846 |
| 3 | 3_V4 | CLEC4A, KLRB1, TNFSF14 | 0,791 | 0,703 | 0,819 | 0,697 | 0,778 | 0,814 |
| 3 | 3_V3 | SIGLEC14, TNFSF14, YOD1 | 0,717 | 0,703 | 0,777 | 0,721 | 0,778 | 0,827 |
| 3 | 3_V2 | CLEC4A, TNFSF14, YOD1 | 0,771 | 0,703 | 0,823 | 0,689 | 0,778 | 0,803 |
| 3 | 3_V1 | KLRB1, TNFSF14, YOD1 | 0,773 | 0,703 | 0,802 | 0,764 | 0,778 | 0,824 |
| 2 | 2_V10 | CLEC4A, SIGLEC14 | 0,737 | 0,703 | 0,793 | 0,745 | 0,778 | 0,846 |
| 2 | 2_V9 | KLRB1, SIGLEC14 | 0,746 | 0,703 | 0,774 | 0,763 | 0,778 | 0,844 |
| 2 | 2_V8 | CLEC4A, KLRB1 | 0,749 | 0,703 | 0,795 | 0,697 | 0,778 | 0,807 |
| 2 | 2_V7 | SIGLEC14, YOD1 | 0,710 | 0,703 | 0,774 | 0,735 | 0,778 | 0,835 |
| 2 | 2_V6 | CLEC4A, YOD1 | 0,786 | 0,703 | 0,828 | 0,706 | 0,778 | 0,807 |
| 2 | 2_V5 | KLRB1, YOD1 | 0,752 | 0,703 | 0,788 | 0,744 | 0,778 | 0,814 |
| 2 | 2_V4 | SIGLEC14, TNFSF14 | 0,652 | 0,703 | 0,750 | 0,651 | 0,778 | 0,809 |
| 2 | 2_V3 | CLEC4A, TNFSF14 | 0,726 | 0,703 | 0,807 | 0,628 | 0,778 | 0,791 |
| 2 | 2_V2 | KLRB1, TNFSF14 | 0,727 | 0,703 | 0,781 | 0,692 | 0,778 | 0,804 |
| 2 | 2_V1 | TNFSF14, YOD1 | 0,726 | 0,703 | 0,776 | 0,676 | 0,778 | 0,785 |
| 1 | 1_V5 | SIGLEC14 | 0,611 | 0,703 | 0,724 | 0,655 | 0,778 | 0,812 |
| 1 | 1_V4 | CLEC4A | 0,723 | 0,703 | 0,804 | 0,653 | 0,778 | 0,797 |
| 1 | 1_V3 | KLRB1 | 0,693 | 0,703 | 0,735 | 0,678 | 0,778 | 0,790 |
| 1 | 1_V2 | YOD1 | 0,730 | 0,703 | 0,770 | 0,662 | 0,778 | 0,780 |
| 1 | 1_V1 | TNFSF14 | 0,645 | 0,703 | 0,724 | 0,558 | 0,778 | 0,747 |

| **Precision Recall (AUC-PR)** | | | **Discovery cohort** | | | **Validation cohort** | | |
|---|---|---|---|---|---|---|---|---|
| **N(Gene)** | **ID** | **Genes** | **AUC-PR (genset)** | **AUC-PR SOFA** | **Combined AUC-PR** | **AUC-PR (genset)** | **AUC-PR SOFA** | **Combined AUC-PR** |
| 5 | 5_V1 | CLEC4A, KLRB1, SIGLEC14, TNFSF14, YOD1 | 0,415 | 0,164 | 0,443 | 0,419 | 0,330 | 0,528 |
| 4 | 4_V5 | CLEC4A, KLRB1, SIGLEC14, YOD1 | 0,369 | 0,164 | 0,440 | 0,349 | 0,330 | 0,491 |
| 4 | 4_V4 | CLEC4A, KLRB1, SIGLEC14, TNFSF14 | 0,392 | 0,164 | 0,457 | 0,306 | 0,330 | 0,439 |
| 4 | 4_V3 | CLEC4A, SIGLEC14, TNFSF14, YOD1 | 0,341 | 0,164 | 0,367 | 0,308 | 0,330 | 0,497 |
| 4 | 4_V2 | KLRB1, SIGLEC14, TNFSF14, YOD1 | 0,362 | 0,164 | 0,395 | 0,379 | 0,330 | 0,515 |
| 4 | 4_V1 | CLEC4A, KLRB1, TNFSF14, YOD1 | 0,473 | 0,164 | 0,357 | 0,426 | 0,330 | 0,497 |
| 3 | 3_V10 | CLEC4A, KLRB1, SIGLEC14 | 0,340 | 0,164 | 0,423 | 0,287 | 0,330 | 0,428 |
| 3 | 3_V9 | CLEC4A, SIGLEC14, YOD1 | 0,311 | 0,164 | 0,372 | 0,285 | 0,330 | 0,454 |
| 3 | 3_V8 | KLRB1, SIGLEC14, YOD1 | 0,317 | 0,164 | 0,409 | 0,341 | 0,330 | 0,473 |
| 3 | 3_V7 | CLEC4A, KLRB1, YOD1 | 0,455 | 0,164 | 0,343 | 0,360 | 0,330 | 0,448 |
| 3 | 3_V6 | CLEC4A, SIGLEC14, TNFSF14 | 0,294 | 0,164 | 0,371 | 0,225 | 0,330 | 0,422 |
| 3 | 3_V5 | KLRB1, SIGLEC14, TNFSF14 | 0,343 | 0,164 | 0,374 | 0,287 | 0,330 | 0,430 |
| 3 | 3_V4 | CLEC4A, KLRB1, TNFSF14 | 0,381 | 0,164 | 0,351 | 0,278 | 0,330 | 0,372 |
| 3 | 3_V3 | SIGLEC14, TNFSF14, YOD1 | 0,304 | 0,164 | 0,300 | 0,287 | 0,330 | 0,472 |
| 3 | 3_V2 | CLEC4A, TNFSF14, YOD1 | 0,374 | 0,164 | 0,342 | 0,388 | 0,330 | 0,469 |
| 3 | 3_V1 | KLRB1, TNFSF14, YOD1 | 0,381 | 0,164 | 0,316 | 0,419 | 0,330 | 0,476 |
| 2 | 2_V10 | CLEC4A, SIGLEC14 | 0,307 | 0,164 | 0,349 | 0,232 | 0,330 | 0,381 |
| 2 | 2_V9 | KLRB1, SIGLEC14 | 0,331 | 0,164 | 0,360 | 0,270 | 0,330 | 0,432 |
| 2 | 2_V8 | CLEC4A, KLRB1 | 0,363 | 0,164 | 0,356 | 0,254 | 0,330 | 0,370 |
| 2 | 2_V7 | SIGLEC14, YOD1 | 0,251 | 0,164 | 0,329 | 0,271 | 0,330 | 0,430 |
| 2 | 2_V6 | CLEC4A, YOD1 | 0,313 | 0,164 | 0,284 | 0,310 | 0,330 | 0,431 |
| 2 | 2_V5 | KLRB1, YOD1 | 0,357 | 0,164 | 0,275 | 0,375 | 0,330 | 0,453 |
| 2 | 2_V4 | SIGLEC14, TNFSF14 | 0,253 | 0,164 | 0,327 | 0,191 | 0,330 | 0,358 |
| 2 | 2_V3 | CLEC4A, TNFSF14 | 0,281 | 0,164 | 0,302 | 0,183 | 0,330 | 0,387 |
| 2 | 2_V2 | KLRB1, TNFSF14 | 0,304 | 0,164 | 0,278 | 0,245 | 0,330 | 0,366 |
| 2 | 2_V1 | TNFSF14, YOD1 | 0,273 | 0,164 | 0,232 | 0,308 | 0,330 | 0,454 |
| 1 | 1_V5 | SIGLEC14 | 0,236 | 0,164 | 0,293 | 0,192 | 0,330 | 0,347 |
| 1 | 1_V4 | CLEC4A | 0,253 | 0,164 | 0,292 | 0,192 | 0,330 | 0,346 |
| 1 | 1_V3 | KLRB1 | 0,278 | 0,164 | 0,244 | 0,194 | 0,330 | 0,342 |
| 1 | 1_V2 | YOD1 | 0,191 | 0,164 | 0,201 | 0,300 | 0,330 | 0,436 |
| 1 | 1_V1 | TNFSF14 | 0,200 | 0,164 | 0,203 | 0,135 | 0,330 | 0,288 |

| | | | | | | | | |
|---|---|---|---|---|---|---|---|---|
| AUC and AUC-PR quantify prediction accuracy of cEP using expression data from day of admission, only. Abbreviations: AUC: area under the curve; AUC-PR: area under the curve for precisicion recall curves. | | | | | | | | |

### Discussion

Using an unbiased approach, including discovery and validation cohorts of 455 hospitalized CAP patients, the inventors identified a 5-gene-expression-signature (STYCK) predicting the disease course in CAP patients. This gene expression score was superior to conventional metrics, such as C(U)RB-65 score, CRP or PCT levels in predicting an unfavorable course in hospitalized patients with CAP.

By evaluating whole blood gene expression data with an internal matched validation cohort, a new 5-gene-based score (the PROGRESS-STYCK signature) was identified that can predict a severe disease course in CAP patients. The transcriptomic biomarker outperformed a broad range of available clinical and serological markers, which all failed to achieve a reasonable prediction performance of the endpoint combining need for organ support, such as mechanical ventilation, dialysis or even mortality. In addition, adding the transcriptomic biomarker ("SOFA+") also improved the performance of the SOFA score, a clinical score that was superior to other routine metrics and scores to indicate severe course of CAP including need for ICU admission in a prior study (Ahnert (2019)). All five identified genes for building the score are involved in the regulation of adaptive or innate immunity. Except for KLRB1, none of the transcripts has been previously associated with CAP. The genes were either significantly upregulated (YOD1) or downregulated (CLEC4A, KLRB1, TNFSF14, SIGLEC14). The combination of the expression of the five genes combined in the STYCK score provided a prediction for disease severity that was superior to conventional markers and scores.

The obtained STYCK score can be used independently of additional clinical information to predict the cEP, but performance is further improved when combined with the SOFA score ("SOFA+"). The measurements of 5 mRNAs from whole blood can be implemented at the bedside as a point-of-care test. This allows for a better and rapid stratification of CAP patients upon admission.

### References

Ahnert P, Creutz P, Scholz M, et al. PROGRESS - prospective observational study on hospitalized community acquired pneumonia. BMC Pulm Med 2016; 16: 108. https://doi.org/10.1186/s12890-016-0255-8.

Ahnert P, Creutz P, Horn K, et al. Sequential organ failure assessment score is an excellent operationalization of disease severity of adult patients with hospitalized community acquired pneumonia - results from the prospective observational PROGRESS study. Crit Care 2019; 23: 110. https://doi.org/10.1186/sl3054-019-2316-x.

Almansa R, Heredia-Rodríguez M, Gomez-Sanchez E, et al. Transcriptomic correlates of organ failure extent in sepsis. J Infect 2015; 70: 445-56. https://doi.org/10.1016/j.jinf.2014.12.010.

Bauer M, Giamarellos-Bourboulis EJ, Kortgen A, et al. ATranscriptomic Biomarker to Quantify Systemic Inflammation in Sepsis - A Prospective Multicenter Phase II Diagnostic Study. EBioMedicine 2016; 6: 114-25. https://doi.org/10.1016/j.ebiom.2016.03.006.

Bray NL, Pimentel H, Melsted P, Pachter L. Near-optimal probabilistic RNA-seq quantification. Nat Biotechnol 2016; 34: 525-27. https://doi.org/10.1038/nbt.3519.

Cohen Freue GV, Hollander Z, Shen E, et al. MDQC: a new quality assessment method for microarrays based on quality control reports. Bioinformatics 2007; 23: 3162-69. https://doi.org/10.1093/bioinformatics/btm487.

Committee for Human Medicinal Products. ICH E6 (R2) Good clinical practice. Step 5. https://www.ema.europa.eu/en/documents/scientific-guideline/ich-e-6-r2-guideline-good-clinical-practice-step-5_en.pdf (accessed Feb 05, 2020).

Daley T, Smith AD. Predicting the molecular complexity of sequencing libraries. Nat Methods 2013; 10: 325-27. https://doi.org/10.1038/nmeth.2375.

Dela Cruz CS, Wunderink RG, Christiani DC, et al. Future Research Directions in Pneumonia. NHLBI Working Group Report. Am J Respir Crit Care Med 2018; 198: 256-63. https://doi.org/10.1164/rccm.201801-0139WS.

Du P, Kibbe WA, Lin SM. lumi: a pipeline for processing Illumina microarray. Bioinformatics 2008; 24: 1547-48. https://doi.org/10.1093/bioinformatics/btn224.

Ewels P, Magnusson M, Lundin S, Käller M. MultiQC: summarize analysis results for multiple tools and samples in a single report. Bioinformatics 2016; 32: 3047-48. https://doi.org/10.1093/bioinformatics/btw354.

Gearhart AM, Furmanek S, English C, Ramirez J, Cavallazzi R. Predicting the need for ICU admission in community-acquired pneumonia. Respir Med 2019; 155: 61- 65. https://doi.org/10.1016/j.rmed.2019.07.007.

Jain S, Self WH, Wunderink RG, et al. Community-Acquired Pneumonia Requiring Hospitalization among U.S. Adults. N Engl J Med 2015; 373: 415-27. https://doi.org/10.1056/NEJMoa1500245.

Johnson WE, Li C, Rabinovic A. Adjusting batch effects in microarray expression data using empirical Bayes methods. Biostatistics 2007; 8: 118-27. https://doi.org/10.1093/biostatistics/kxj037.

Kämpf C, Specht M, Scholz A, et al. uap: reproducible and robust HTS data analysis. BMC Bioinformatics 2019; 20: 664. https://doi.org/10.1186/s12859-019-3219-1.

Kim D, Paggi JM, Park C, Bennett C, Salzberg SL. Graph-based genome alignment and genotyping with HISAT2 and HISAT-genotype. Nat Biotechnol 2019; 37: 907-15. https://doi.org/10.1038/s41587-019-0201-4.

Kirsten H, Al-Hasani H, Holdt L, et al. Dissecting the genetics of the human transcriptome identifies novel trait-related trans-eQTLs and corroborates the regulatory relevance of non-protein coding loci†. Hum Mol Genet 2015; 24: 4746-63. https://doi.org/10.1093/hmg/ddv194.

Kolditz M, Tesch F, Mocke L, Höffken G, Ewig S, Schmitt J. Burden and risk factors of ambulatory or hospitalized CAP: A population based cohort study. Respir Med 2016; 121: 32-38. https://doi.org/10.1016/j.rmed.2016.10.015.

Krämer A, Green J, Pollard J, Tugendreich S. Causal analysis approaches in Ingenuity Pathway Analysis. Bioinformatics 2014; 30: 523-30. https://doi.org/10.1093/bioinformatics/btt703.

Langmead B, Salzberg SL. Fast gapped-read alignment with Bowtie 2. Nat Methods 2012; 9: 357-59. https://doi.org/10.1038/nmeth.1923.

Love MI, Huber W, Anders S. Moderated estimation of fold change and dispersion for RNA- seq data with DESeq2. Genome Biol 2014; 15: 550. https://doi.org/10.1186/s13059-014-0550-8.

Newman AM, Liu CL, Green MR, et al. Robust enumeration of cell subsets from tissue expression profiles. Nat Methods 2015; 12: 453-57. https://doi.org/10.1038/nmeth.3337.

Othmer Kirk, Encyclopedia of chemical technology, 4th ed., executive editor, Kroschwitz JI; editor, M. Howe-Grant, John Wiley & Sons, 1993, vol.15, p. 518-562, incorporated herein by reference, including citations on pages 551-562.

Ramírez P, Ferrer M, Martí V, et al. Inflammatory biomarkers and prediction for intensive care unit admission in severe community-acquired pneumonia. Crit Care Med 2011; 39: 2211-17. https://doi.org/10.1097/CCM.0b013e3182257445.

Renaud B, Brun-Buisson C, Santin A, et al. Outcomes of early, late, and no admission to the intensive care unit for patients hospitalized with community-acquired pneumonia. Acad Emerg Med 2012; 19: 294-303. https://doi.org/10.1111/j.1553-2712.2012.01301.x.

Restrepo MI, Mortensen EM, Rello J, Brody J, Anzueto A. Late admission to the ICU in patients with community-acquired pneumonia is associated with higher mortality. Chest 2010; 137: 552-57. https://doi.org/10.1378/chest.09-1547.

Ritchie ME, Phipson B, Di Wu, et al. limma powers differential expression analyses for RNA-sequencing and microarray studies. Nucleic Acids Res 2015; 43: e47. https://doi.org/10.1093/nar/gkv007.

Robin X, Turck N, Hainard A, et al. pROC: an open-source package for R and S+ to analyze and compare ROC curves. BMC Bioinformatics 2011; 12: 77. https://doi.org/10.1186/1471-2105-12-77.

Schubert M, Lindgreen S, Orlando L. AdapterRemoval v2: rapid adapter trimming, identification, and read merging. BMC Res Notes 2016; 9: 88. https://doi.org/10.1186/s13104-016-1900-2.

Scicluna BP, Klein Klouwenberg PMC, van Vught LA, et al. A molecular biomarker to diagnose community-acquired pneumonia on intensive care unit admission. Am J Respir Crit Care Med 2015; 192: 826-35. https://doi.org/10.1164/rccm.201502-03550C.

Scicluna BP, van Vught LA, Zwinderman AH, et al. Classification of patients with sepsis according to blood genomic endotype: a prospective cohort study. The Lancet Respiratory Medicine 2017; 5: 816-26. https://doi.org/10.1016/S2213-2600(17)30294-1.

Sibila O, Restrepo MI. Biomarkers in community-acquired pneumonia: still searching for the one. Eur Respir J 2019; 53. https://doi.org/10.1183/13993003.02469-2018.

Soneson C, Love MI, Robinson MD. Differential analyses for RNA-seq: transcript-level estimates improve gene-level inferences. F1000Res 2015; 4: 1521. https://doi.org/10.12688/f1000research.7563.2.

Strimmer K. fdrtool: a versatile R package for estimating local and tail area-based false discovery rates. Bioinformatics 2008; 24: 1461-62. https://doi.org/10.1093/bioinformatics/btn209.

Sweeney TE, Shidham A, Wong HR, Khatri P. A comprehensive time-course-based multicohort analysis of sepsis and sterile inflammation reveals a robust diagnostic gene set. Sci Transl Med 2015; 7: 287ra71. https://doi.org/10.1126/scitranslmed.aaa5993.

Sweeney TE, Wong HR, Khatri P. Robust classification of bacterial and viral infections via integrated host gene expression diagnostics. Sci Transl Med 2016; 8: 346ra91. https://doi.org/10.1126/scitranslmed.aaf7165.

Sweeney TE, Haynes WA, Vallania F, loannidis JP, Khatri P. Methods to increase reproducibility in differential gene expression via meta-analysis. Nucleic Acids Res 2017; 45: e1. https://doi.org/10.1093/nar/gkw797.

Sweeney TE, Perumal TM, Henao R, et al. A community approach to mortality prediction in sepsis via gene expression analysis. Nat Commun 2018; 9: 694. https://doi.org/10.1038/s41467-018-03078-2.

Torres A, Chalmers JD, Dela Cruz CS, et al. Challenges in severe community- acquired pneumonia: a point-of-view review. Intensive Care Med 2019; 45: 159-71. https://doi.org/10.1007/s00134-019-05519-y.

Tsalik EL, Langley RJ, Dinwiddie DL, et al. An integrated transcriptome and expressed variant analysis of sepsis survival and death. Genome Med 2014; 6: 111. https://doi.org/10.1186/s13073-014-0111-5. van Vught LA, Scicluna BP, Wiewel MA, et al. Comparative Analysis of the Host Response to Community-acquired and Hospital-acquired Pneumonia in Critically III Patients. Am J Respir Crit Care Med 2016; 194: 1366-74. https://doi.org/10.1164/rccm.201602-0368OC.

Wang L, Wang S, Li W. RSeQC: quality control of RNA-seq experiments. Bioinformatics 2012; 28: 2184-85. https://doi.org/10.1093/bioinformatics/bts356.

Wingett SW, Andrews S. FastQ Screen: A tool for multi-genome mapping and quality control. F1000Res 2018; 7: 1338. https://doi.org/10.12688/f1000research.15931.2.

Wong HR, Cvijanovich NZ, Anas N, et al. Developing a clinically feasible personalized medicine approach to pediatric septic shock. Am J Respir Crit Care Med 2015; 191: 309-15. https://doi.org/10.1164/rccm.201410-1864OC.

World Medical Association I. WMA Declaration of Helsinki - Ethical Principles for Medical Research Involving Human Subjects - WMA - The World Medical Association. https://www.wma.net/policies-post/wma-declaration-of-helsinki-ethical-principles-for-medical-research-involving-human-subjects/ (accessed Jan 03, 2020).

### Sequence listing

The sequences of the sequence listing are filed separately. Below table 7 discloses the Ensembl transcript IDs, which are represented by the individual SEQ ID Nos, further the table lists the probe sequences which were used for detection of the biomarkers.

**Table 7: Ensembl transcript variants accession numbers of the biomarkers of the invention and corresponding SEQ ID NO; further listed are probe sequences which were used for detection of the selected biomarkers on Illumina HT12-v4 Chip**

| **SEQ ID NO** | **Biomarker** | **Ensembl transcript accession numbers** |
|---|---|---|
| 1 | TNFSF14 (ENSG00000125735) | ENST00000245912.7 |
| 2 | | ENST00000599359.1 |
| 3 | | ENST00000675206.1 |
| 4 | YOD1 (ENSG00000180667) | ENST00000315927.9 |
| 5 | | ENST00000367084.1 |
| 6 | KLRB1 (ENSG00000111796) | ENST00000229402.4 |
| 7 | CLEC4A (ENSG00000111729) | ENST00000345999.9 |
| 8 | | ENST00000352620.9 |
| 9 | | ENST00000360500.5 |
| 10 | | ENST00000229332.12 |
| 11 | | ENST00000641376.1 |
| 12 | SIGLEC14 (ENSG00000254415) | ENST00000360844.7 |
| 13 | | ENST00000533866.1 |

| | **Biomarker** | **Probe sequence** |
|---|---|---|
| 14 | TNFSF14 | GGGTCTGACACGTGGAGAACTCAGAGGGTGCCTCAGGGGAAAGAAAACTC |
| 15 | YOD1 | CAGTTTTCCAGTCACATTGGTGCCATTCAGGACTCCAGCTGTTTACAGGA |
| 16 | KLRB1 | CAGTCTAGCTGATTGTTCCACCAAAGAATCCAGCCTGCTGCTTATTCGAG |
| 17 | CLEC4A | GAGCATTTTTTCATGTGCCAGAGCCTGTACTGGAGGCCCCCATTGTGCAC |
| 18 | SIGLEC14 | CTGGTCTCCTCAGCTTCCCTATCCATATAGGCACAGAGAGGCAGCATTTG |

## Claims

1. A diagnostic method for the ex-vivo prognosis, prediction or risk stratification for mortality, disease outcome or ICU admission of a patient who has been diagnosed with community acquired pneumonia (CAP) comprising the steps of
a) providing a sample from the patient,
b) analysing the expression level of at least one nucleic acid in the sample,
wherein the patient's prognosis, prediction or risk stratification is determined according to the expression levels of the at least one nucleic acid,
wherein the at least one nucleic acid is selected from the group comprising CLEC4A, KLRB1, SIGLEC14, TNFSF14 and YOD1.

2. The method according to claim 1, wherein the sample is selected from the group comprising blood, whole blood, blood serum, blood plasma, buffy coat, urine, sputum, tracheal secretions, cheek swabs and tissue samples.

3. The method of claim 1, wherein expression level analysis of nucleic acids comprises at least two, preferably at least 3, more preferably at least 4 and most preferably at least 5 nucleic acid, wherein the analysed nucleic acids are preferably selected from the group comprising SEQ ID NO: 1-13.

4. The method according to any one of the preceding claims, wherein the analysis of the expression level is performed according to a method selected from qPCR, qRT-PCR, digital PCR, sequencing, NGS methods, mRNA-Arrays, gene chips and RNA-microarrays.

5. The method according to any one of the preceding claims, wherein the expression levels are normalized to general reference genes and or specific reference genes validated for blood samples.

6. The method according to any one of the preceding claims wherein the method provides a prediction for the likelihood of a composite endpoint consisting of 28-day mortality or ICU admission.

7. The method according to any one of the preceding claims wherein the method further comprises determination of a predictive score.

8. The method of claim 7 wherein the predictive score of the invention is combined with a clinical scores, preferably wherein the clinical score is selected from the groups comprising SOFA: Sequential Organ Failure Assessment Score; Variations of CURB criteria scores (CRB, CRB65, CURB, CURB65); ATS: Australian Triage Scale; PCT: Procalcitonin assay; CRP: C-reactive protein; PSI: Pneumonia severity index; SIRS: Systemic inflammatory response syndrome criteria; qSOFA: quick SOFA (Sequential Organ Failure Assessment); Halm: Halm's criteria; SCAP: Severe Community-Acquired Pneumonia Score; SMART-COP Score (systolic blood pressure, multilobar infiltrates, albumin, respiratory rate, tachycardia, confusion, oxygen, and pH).

9. The method of any one of the preceding claims wherein the method is performed on admission of the patient

10. A nucleic acid that hybridizes under stringent conditions to one of the nucleic acids according to SEQ ID NO: 1-13, or the reverse complement thereof or any part thereof, or a nucleic acid that shares preferably at least 85%, 90%, 95% or 99% sequence identity with one of the sequences of SEQ: 1-13, or the reverse complement thereof or any part thereof

11. The nucleic acid according to claim 10, wherein the nucleic acid is about 10 to 100 nucleotides in length.

12. The nucleic acid according to claim 10 or 11, wherein the nucleic acid comprises a detectable label.

13. Use of a nucleic acid according to any of claims 10 to 12 for the prognosis, prediction or risk stratification for mortality, disease outcome or ICU admission of a patient who has been diagnosed with community acquired pneumonia (CAP).

14. A kit for the prognosis, prediction or risk stratification for mortality, disease outcome or ICU admission of a patient who has been diagnosed with community acquired pneumonia (CAP) comprising
a) a nucleic acid according to SEQ ID NO: 1-13;
b) reagents for nucleic acid amplification and/or quantification and/or detection.
c) optionally standards and/or internal standards and
d) reference data for patients under reference conditions,
e) a manual.
